# EUROPEAN PATENT SPECIFICATION

(11) **EP 4 448 545 B1**
(45) Date of publication and mention of the grant of the patent: **20.08.2025**
(21) Application number: 22835716.6
(22) Date of filing: 12.12.2022
(51) Int. Cl.: C07K 7/08

(54) **MANUFACTURING PROCESS FOR THE PRODUCTION OF LINACLOTIDE**
FERTIGUNGSVERFAHREN ZUR HERSTELLUNG VON LINACLOTID
PROCÉDÉ DE FABRICATION POUR LA PRODUCTION DE LINACLOTIDE

(30) Priority: 13.12.2021 EP 21214095
(43) Date of publication of application: 23.10.2024
(73) Proprietor: Chemi SPA, 20092 Cinisello Balsamo (MI) (IT)
(72) Inventor: ANNONI, Paola, 20092 Cinisello Balsamo (MI) (IT); CAPPELLETTI, Silvana, 20092 Cinisello Balsamo (MI) (IT); CELONA, Alessandra, 20092 Cinisello Balsamo (MI) (IT); SIMONELLI, Barbara, 20092 Cinisello Balsamo (MI) (IT); TURCHETTA, Stefano, 03010 Patrica (FR) (IT); CAPPELLINI, Lorenzo, 20092 Cinisello Balsamo (MI) (IT)
(74) Representative: Dragotti & Associati S.R.L.
(86) International application number: PCT/EP2022/085451
(87) International publication number: WO 2023/110781

(56) References cited:
- WO-A1-2010/059733
- WO-A1-2013/025969
- CN-A- 109 053 863
- LI HAIDI ET AL: "Total Liquid-phase Sythesis, Head-to-tail Cyclization and Synergistic Self-cleavage of Peptide on Small-molecular Supports", 30 June 2021 (2021-06-30), XP055919626, Retrieved from the Internet <URL:https://chemrxiv.org/engage/api-gateway/chemrxiv/assets/orp/resource/item/60db51811a89f2c17358b5a7/original/total-liquid-phase-sythesis-head-to-tail-cyclization-and-synergistic-self-cleavage-of-peptide-on-small-molecular-supports.pdf> [retrieved on 20220510], DOI: 10.1039/x0xx00000x

## Description

### Field of the invention

The present invention relates to a manufacturing process to produce Linaclotide and its acetate salt, more particularly, to obtain amorphous High-Purity Linaclotide or its acetate salt with a chromatographic purity equal to or higher than 99.9% and a content of IMD-Linaclotide and Cys-1-α-ketone-Linaclotide impurities each one lower than 100 ppm, preferably lower than 50 ppm, more preferably lower than 40 ppm.

The invention further relates to an analytical method able to detect IMD-Linaclotide and Cys-1-α-ketone Linaclotide even at 40 ppm level (LOD) and quantify IMD-Linaclotide and Cys-1-α-ketone Linaclotide even at 50 ppm level (LOQ), by lon-Pairing Chromatography (IPC).

### State of the art

Linaclotide acetate is the Drug Substance contained in the commercial products Linzess^{®} for the US market and Constella^{®} for the EU market, in the dosage forms of 72, 145 and 290 mcg capsules.

The Active Pharmaceutical Ingredient is a complex Cys-rich cyclic peptide consisting of 14 aminoacids and containing three regiospecific disulfide bonds between Cys [1-6],[2-10],[5-13] as illustrated in the Formula (I):

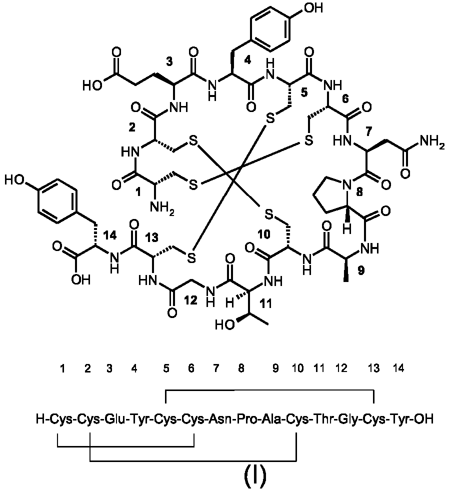

This drug belongs to the class of agonists of guanylate cyclase and acts locally on guanylate cyclase subtype C receptors located in the internal surface of intestinal epithelium. The activation of guanylate cyclase by Linaclotide leads to increased levels of cGMP which in turn activates a secretion of chloride and hydrogenocarbonate into the intestinal lumen, increasing intestinal fluid secretion and accelerating transit.

The structure of Linaclotide was reported for the first time in US7304036, where it is generically described that the production of the peptide can be achieved by fermentation of appropriately modified bacterial vectors or synthetically by Solid Phase Peptide Synthesis (SPPS), but a complete description of the chemical procedure is not given, while the first detailed synthetic method published for Linaclotide is found in Peptide Science 96 (1), 69-80 (2010), where SPPS manufacturing process is reported and oxidative methods for the disulfide bridges formation are proposed.

Chemical preparative procedures of Linaclotide are described, for example, in WO2014188011 (Lonza), where the linear backbone peptide on resin is prepared by SPPS through a one-by-one aminoacid assembling strategy, then the linear peptide is cleaved from the resin and concurrently deprotected; the three disulfide bridges are formed according to random strategy by oxidation with air in dimethylsulphoxide and the crude Linaclotide is then purified by reverse phase chromatography, to be finally isolated in an undisclosed grade of purity by lyophilization from a 50% tert-butanol aqueous solution.

WO2015022575 (Auro Peptides) also describes a chemical synthesis procedure where two fragments are prepared and coupled by a SPPS stepwise strategy to obtain the linear backbone peptide on resin, which is then cleaved from the resin and concurrently deprotected, cyclized by random strategy with air and an oxidizing agent (e.g. hydrogen peroxide) to form the three disulfide bridges and finally purified and lyophilized giving Linaclotide having an HPLC purity, at most, of 98.9%.

WO2016038497 (Auro Peptides) furthermore, reports a method for preparing Linaclotide where SPPS is applied through a one-by-one aminoacid assembling strategy, to generate the linear backbone peptide on resin, which is then freed from the resin and deprotected (concurrently or sequentially), cyclized by random strategy with air and an oxidizing agent (e.g. hydrogen peroxide) and finally purified by reverse phase chromatography and lyophilized to yield a final product having an HPLC purity of 98.9% or a generic HPLC purity of > 99%, without details of the structure of the impurities present in the product.

WO2017101810 (Hybio Pharmaceuticals), describes a regioselective synthesis of Linaclotide consisting in the preliminary preparation of the linear backbone peptide on resin by SPPS via one-by-one aminoacid assembling strategy, the oxidative formation of the first disulfide bridge [1-6] on the peptide still linked to the resin, the oxidative formation of the second disulfide bridge [2-10] in solution, and finally the oxidative formation of the third disulfide bridge [5-13] after deprotection of the two methylated cysteines.

Crude Linaclotide is purified by reverse phase chromatography and then lyophilized. Although it is described that the synthesis proceeds in a regioselective way, the oxidative nature of the reactions used to create the necessary disulfide bonds does not allow to avoid the formation of dimers and multimer impurities of the peptide. In addition, the use of hardly industrially adequate solvents such as diethyl ether is also described, as well as an HPLC purity of the final Linaclotide of 99.5%, with no mention of the content for example of the IMD-Linaclotide and Cys-1-α-ketone-Linaclotide impurities. However, an additional purification by reverse phase chromatography of the intermediate bis-disulfide peptide is described.

WO2017134687 (Cipla) also describes a preparation of Linaclotide by initial manufacturing of the linear backbone peptide on resin by SPPS via one-by-one aminoacid assembling strategy, then a concurrent cleavage from the resin and removal of the S-phenylacetamidomethyl protecting groups follow, to finally oxidize the product in aqueous solvent via random strategy, purify it by reverse phase chromatography and lyophilize it to generate a final product having a generic HPLC purity of > 99%, free from dimers and multimer impurities.

WO2019113872 (Shenzhen) details as well a manufacturing method of Linaclotide through the preparation of the linear backbone peptide on resin either by SPPS via one-by-one aminoacid assembling strategy or by stepwise strategy, which once obtained is not cleaved from the resin but cyclized by oxidation via N-alogenyl sucinimmide; crude Linaclotide on resin is then detached, purified by reverse phase chromatography and lyophilized.

CN 109 053 863 A (HANGZHOU TAIJIA BIOTECH) discloses amorphous Linaclotide with a very high purity of up to 99.8 %.

WO 2013/025969 A1 (IRONWOOD PHARMACEUTICALS) discloses a method for detecting and quantifying the impurity content of Cys-1-alpha-ketone in the product Linaclotide, wherein the content and purity of Linaclotide may be determined by reverse phase gradient liquid chromatography.

With reference to processes for the preparation of Linaclotide used in the state of the art, the inventors have observed that these processes do not provide amorphous Linaclotide having high purity of at least 99.9% and a content of IMD-Linaclotide and Cys-1-α-ketone-Linaclotide impurities each one lower than 100 ppm, preferably lower than 50 ppm, more preferably lower than 40 ppm.

The present inventors have unexpectedly found a simplified, industrially applicable and robust process for preparing Linaclotide and its acetate salt, wherein the peptide is totally prepared by Liquid Phase Peptide Synthesis (LPPS) and without any intermediate purification. More particularly, the method allows to produce an amorphous Linaclotide or its acetate salt characterized by chromatographic purity equal to or higher than 99.9% and particularly having a content of IMD-Linaclotide and Cys-1-α-ketone-Linaclotide impurities each one lower than 100 ppm, preferably lower than 50 ppm (LOQ of the lon-Pairing analytical method) and more preferably lower than 40 ppm (LOD of the lon-Pairing analytical method).

### Abbreviations

ACN: acetonitrile
AcOH: acetic acid
DCHA: dicyclohexylamine
DCM: dichloromethane
DEA: diethylamine
DIPE: diisopropylether
DMF: N,N-dimethylformamide
DSC: Differential Scanning Calorimetry
EDC*HCl: N-(3-Dimethylaminopropyl)-N'-ethylcarbodiimide hydrochloride
Fmoc: Fluorenylmethoxycarbonyl
GPC: Gel Permeation Chromatography
IPA: isopropanol
iPrOAc: isopropylacetate
IPC: Ion-Pairing Chromatography
LOD: Limit of Detection
LOQ: Limit of Quantification
LPPS: Liquid Phase Peptide Synthesis
MTBE: methyl-tert-butylether
NMM: N-methylmorpholine
NMP: N-methylpyrrolidone
OAII: allylester
OtBu: tert-butylester
Oxyma: ethyl cyano(hydroxyimino)acetate
Oxyma-B: 5-(hydroxyimino)-1,3-dimethylpyrimidine-2,4,6(1H,3H,5H)-trione
Pd/C: Palladium on Carbon
Pd(PPh₃)₄: tetrakis(triphenylphosphine)-palladium(0)
PhSiH₃: phenylsilane
PSD: Particle Size Distribution
PyOxim: ethyl cyano(hydroxyimino)acetato-O2tri-1-pyrrolidinylphosphonium hexafluorophosphate
RP-HPLC: Reverse Phase High Performance Liquid Chromatography
RP-HPSD: Reverse Phase High Performance Sample Displacement
tBu: tert-butyl
tBuOH: tert-butanol
TFA: trifluoroacetic acid
TIS: triisopropylsilane
TOTU: O-(Ethoxycarbonyl)cyanomethylenamino-N,N,NÆ,NÆ-tetramethyl-uronium tetrafluoroborate
Trt: Trityl, Triphenylmethyl
PXRD: Powder X-Ray Diffraction
[Ψ(Dmp,H)pro]: dimethoxyphenyl-pseudoprolines

### Definitions

Unless otherwise defined, all terms of art, notations and other scientific terminology used herein are intended to have the meanings commonly understood by those of skill in the art to which this disclosure pertains. In some cases, terms with commonly understood meanings are defined herein for clarity and/or for ready reference; thus, the inclusion of such definitions herein should not be construed to represent a substantial difference over what is generally understood in the art.

The terms "approximately" and "about" herein refer to the range of the experimental error, which may occur in a measurement.

The term "High-Purity" herein refer to a chromatographic purity equal to or higher than 99.9% and a content of IMD-Linaclotide and Cys-1-α-ketone-Linaclotide impurities each one lower than 100 ppm, preferably lower than 50 ppm, more preferably lower than 40 ppm.

The terms "HPLC purity" or "chromatographic purity" herein refers to area under the curve in the chromatogram.

References herein to percent (%) purity are based on chromatographic purity (chromatographic area%).

The terms "comprising", "having", "including" and "containing" are to be construed open-ended terms (i.e. meaning "including, but not limited to") and are to be considered as providing support also for terms as "consist essentially of", "consisting essentially of", "consist of" or "consisting of".

The terms "consist essentially of", "consisting essentially of" are to be construed as semi-closed terms, meaning that no other ingredients which materially affects the basic and novel characteristics of the invention are included (optional excipients may thus included).

The terms "consists of", "consisting of" are to be construed as closed terms.

### Summary of the invention

In a first aspect, the present invention relates to a liquid-phase process for preparing Linaclotide or its acetate salt comprising the following generic steps:
1. an initial phase of construction of the linear protected peptide,
2. a phase of deprotection,
3. a phase of formation of the disulfide bridges to produce crude Linaclotide,
4. optionally, a phase of purification and
5. optionally, a phase of isolation of amorphous High-Purity Linaclotide.

This process is a notable improvement with respect to the prior art and its advantages are summarized below:
1. the same standardized sequence of process operations is cyclically repeated to obtain the protected linear peptide;
2. the protected linear peptide is totally prepared by Liquid Phase Peptide Synthesis (LPPS);
3. no chromatographic purification of any intermediate is executed during the chemical synthesis of the linear protected peptidic backbone as well as of the linear advanced intermediate;
4. the formation of the disulfide bridges is carried out by a non-oxidative method, with a cyclization molar yield of about 70%;
5. the optional purification is preferably performed by a combination of 2 techniques, consisting of preparative Reverse Phase High Performance Sample Displacement (RP-HPSD) and preparative Reverse Phase High Performance Liquid Chromatography (RP-HPLC) with an overall chromatographic purification yield of about 55%;
6. the optional final phase of isolation of High-Purity Linaclotide is performed by lyophilization of either a solution of Linaclotide in t-BuOH-water or of an aqueous suspension of Linaclotide;
7. the final High-Purity Linaclotide consists of an amorphous lyophilized powder.

According to a second aspect thereof, the present invention relates to amorphous Linaclotide or its acetate salt having a chromatographic purity equal to or higher than 99.9% and an amount of any of the following impurities lower than 100 ppm, preferably lower than 50 ppm, more preferably lower than 40 ppm:
- IMD-Linaclotide (Impurity 1);
- Cys-1-α-ketone (Impurity 2).

Preferably, the Linaclotide or its acetate salt has a content of multimers equal to or lower than 0.1% area%.

According to a third aspect thereof, the present invention relates to an analytical lon-Pairing Chromatography (IPC) method to analyze Linaclotide or its acetate salt, which is able to detect (with a LOD 40 ppm) and quantify (with LOQ 50 ppm) trace quantities of the impurities IMD-Linaclotide and Cys-1-α-ketone-Linaclotide in the amorphous High-Purity Linaclotide. The structures of IMD-Linaclotide and Cys-1-α-ketone-Linaclotide impurities are reported below:

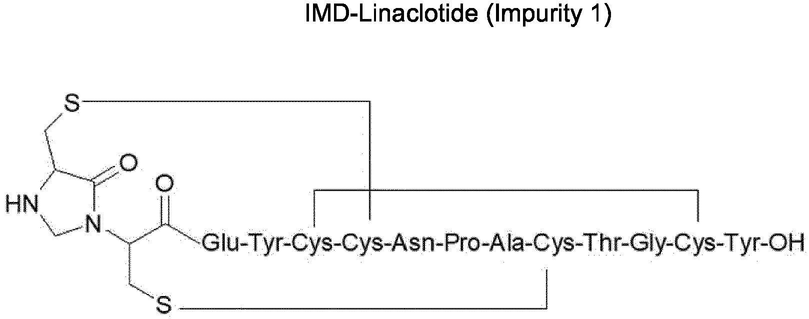

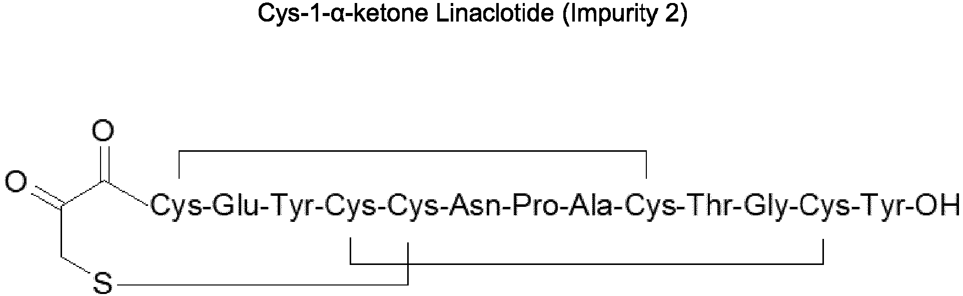

### Description of the figures:

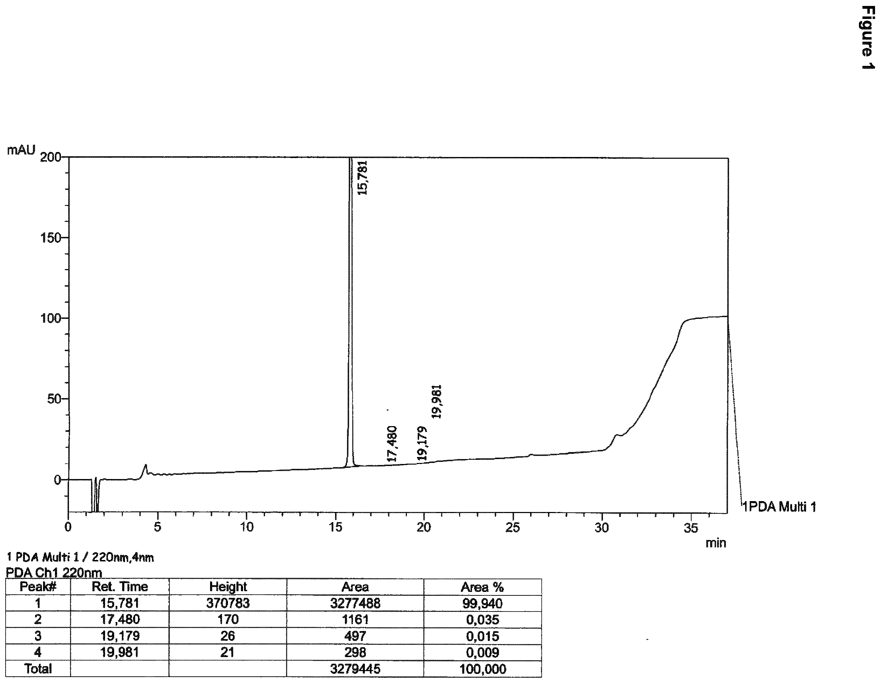

- **Figure 1:**: lon-Pairing chromatogram (for evaluation of Chromatographic Purity) of amorphous High-Purity Linaclotide obtained by MANUFACTURING OPTION 1 (Chromatographic Purity: 99.94%).

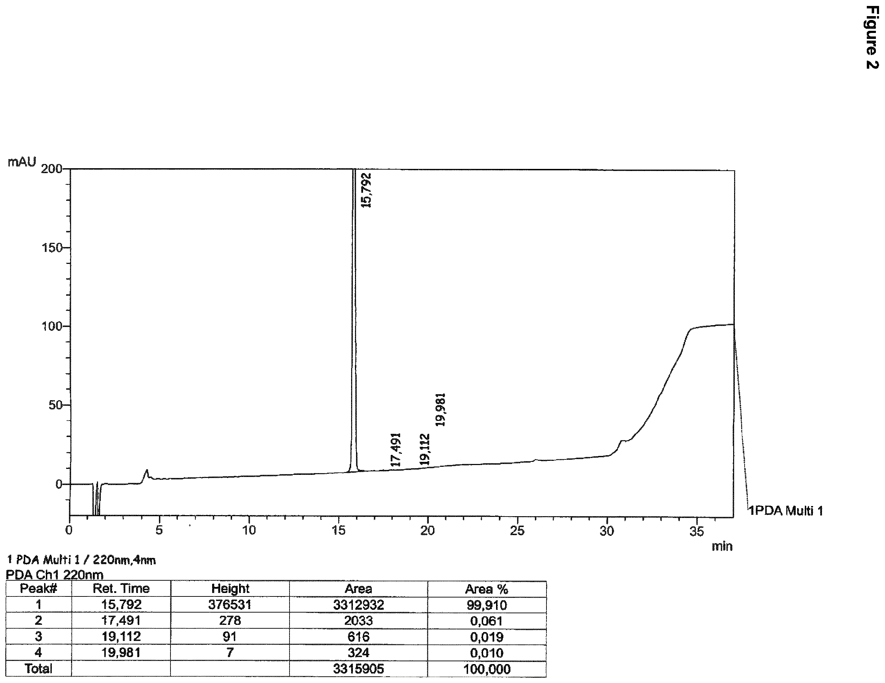

- **Figure 2:**: lon-Pairing chromatogram (for evaluation of Chromatographic Purity) of amorphous High-Purity Linaclotide obtained by MANUFACTURING OPTION 2 (Chromatographic Purity: 99.91%).

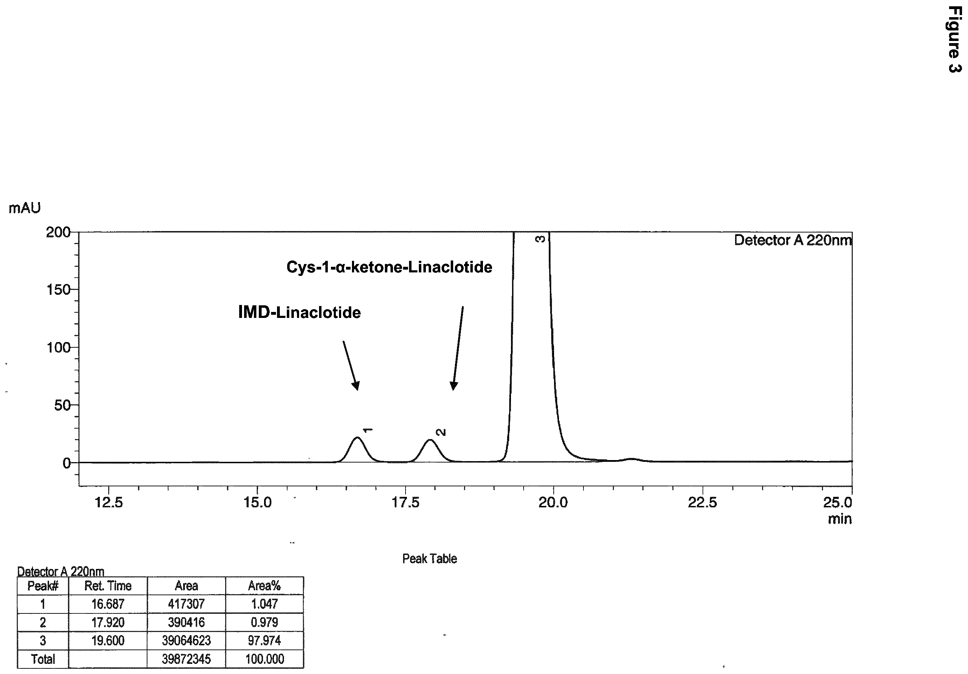

- **Figure 3:**: lon-Pairing chromatogram of spiked Linaclotide with IMD-Linaclotide and Cys-1-α-ketone-Linaclotide impurities (IMD-Linaclotide RRT 0.84-0.85 1.0% w/w; Cys-1-α-ketone-Linaclotide RRT 0.91-0.92 1.0% w/w).

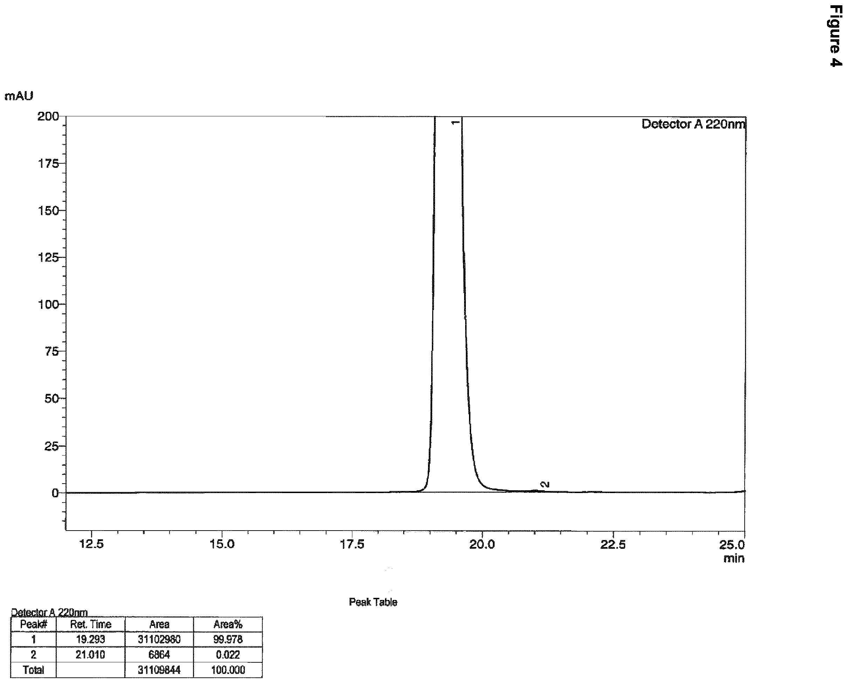

- **Figure 4:**: lon-Pairing chromatogram (for the evaluation of IMD-Linaclotide and Cys-1-α-ketone-Linaclotide content) of amorphous High-Purity Linaclotide obtained by MANUFACTURING OPTION 1; IMD-Linaclotide RRT 0.84-0.85: LT 40 ppm (LT LOD); Cys-1-α-ketone-Linaclotide RRT 0.91-0.92: LT 40 ppm (LT LOD).

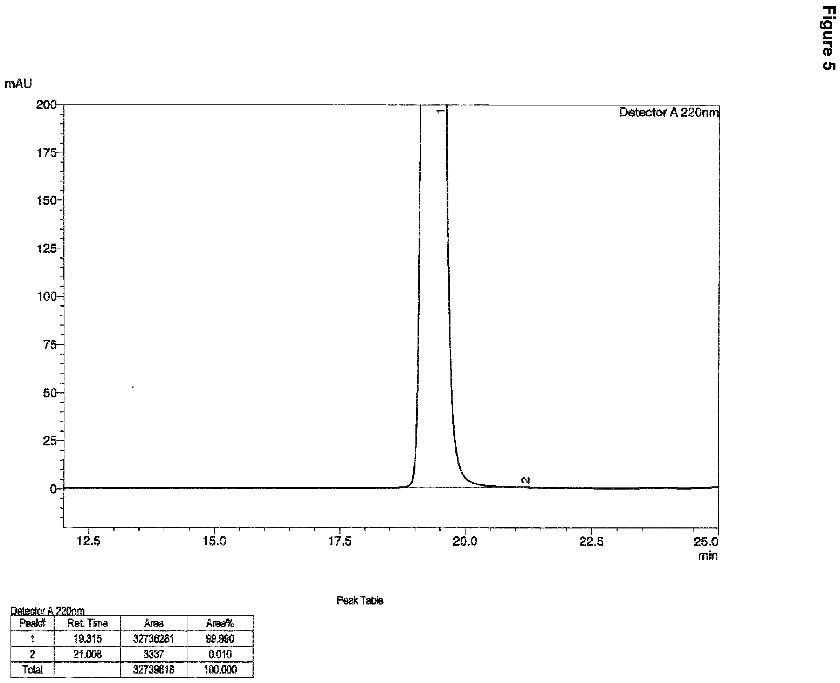

- **Figure 5:**: lon-pairing chromatogram (for the evaluation of IMD-Linaclotide and Cys-1-α-ketone-Linaclotide content) of amorphous High-Purity Linaclotide obtained by MANUFACTURING OPTION 2; IMD-Linaclotide RRT 0.84-0.85: LT 40 ppm (LT LOD); Cys-1-α-ketone-Linaclotide RRT 0.91-0.92: LT 40 ppm (LT LOD)

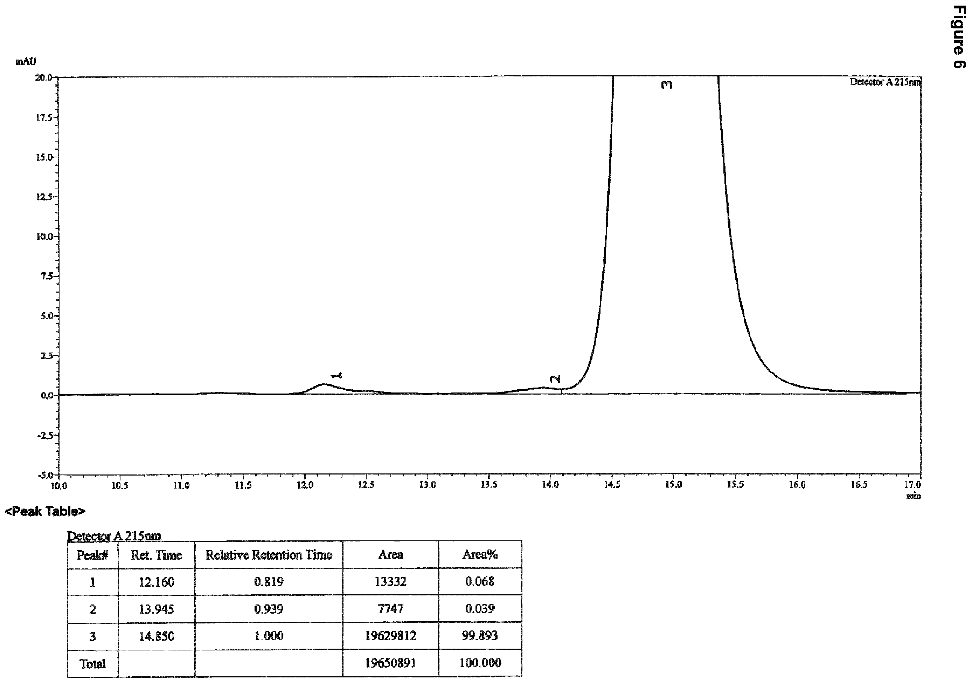

- **Figure 6:**: GPC chromatogram (for the evaluation of multimers content) of amorphous High-Purity Linaclotide obtained by MANUFACTURING OPTION 1 ; Multimers (Sum of the peaks) RRT ≤ 0.81-0.82: 0.07%.

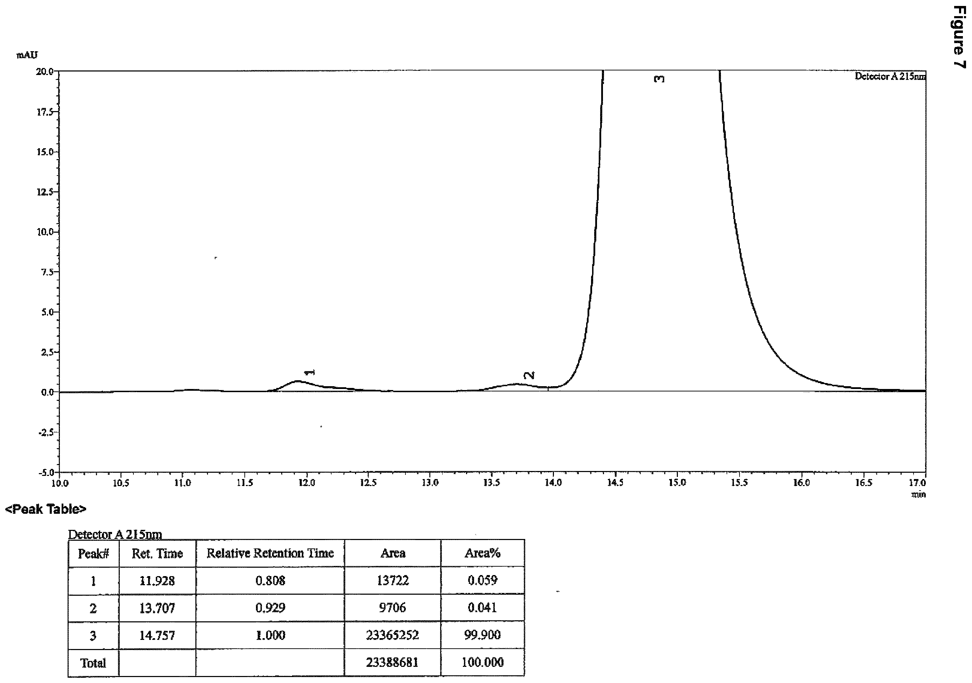

- **Figure 7:**: GPC chromatogram (for the evaluation of multimers content) of amorphous High-Purity Linaclotide obtained by MANUFACTURING OPTION 2; Multimers (Sum of the peaks) RRT ≤ 0.81-0.82: 0.06%.

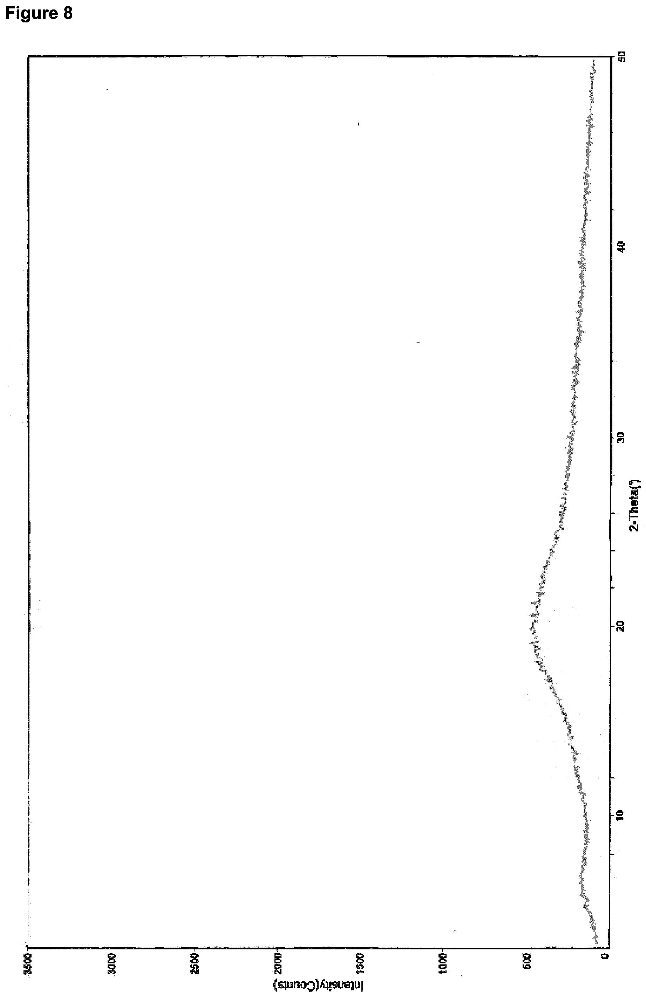

- **Figure 8:**: Powder X-Ray Diffraction (PXRD) of amorphous High-Purity Linaclotide obtained by MANUFACTURING OPTION 1.

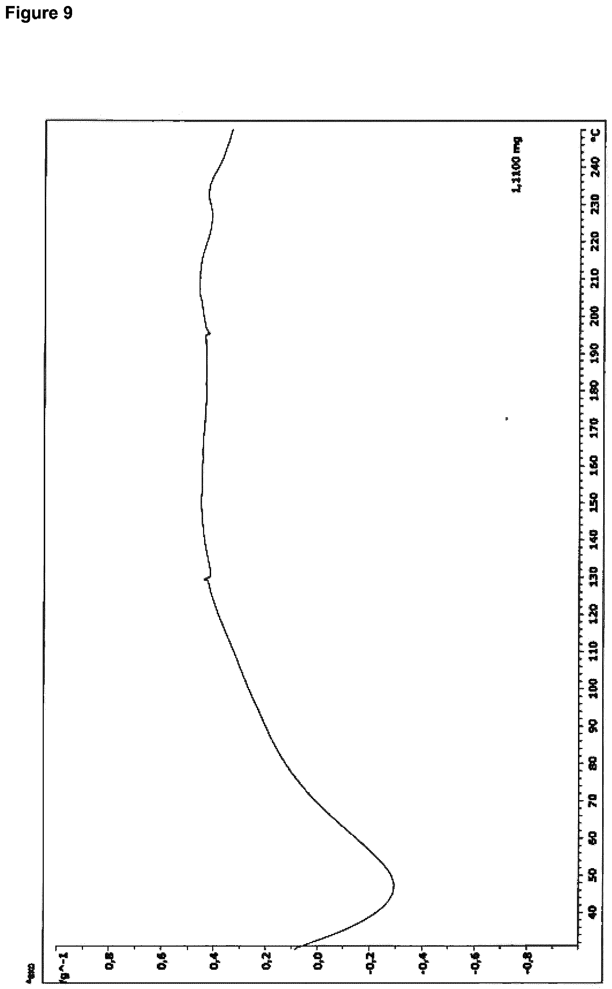

- **Figure 9:**: Differential Scanning Calorimetry (DSC) curve of amorphous High-Purity Linaclotide obtained by MANUFACTURING OPTION 1; Loss of water-solvent in the range 30-100°C, no further endo/exothermic events related to crystalline forms are registered until 250°C.

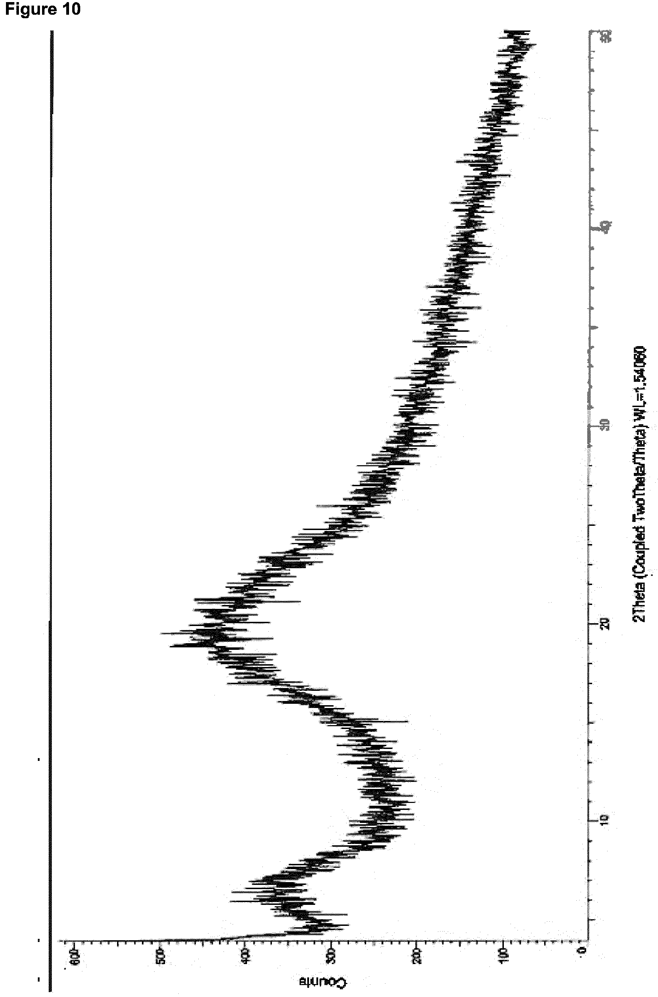

- **Figure 10:**: Powder X-Ray Diffraction (PXRD) of amorphous High-Purity Linaclotide obtained by MANUFACTURING OPTION 2.

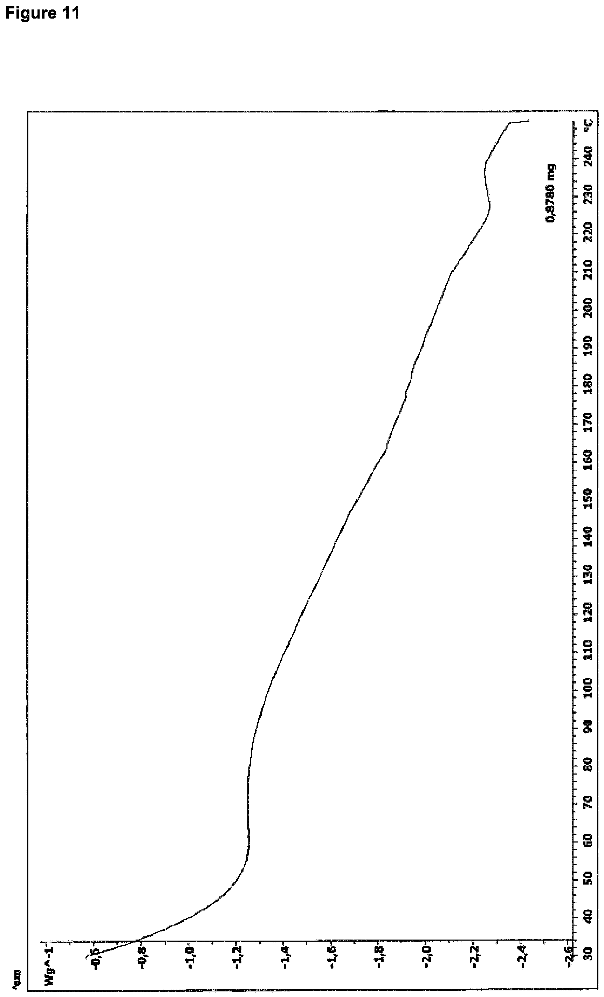

- **Figure 11:**: Differentials Scanning Calorimetry (DSC) curve of amorphous High-Purity Linaclotide obtained by MANUFACTURING OPTION 2; No particular endo/exothermic events related to crystalline forms are registered until 250°C.

### Detailed description of the invention

An object of the present invention is a liquid-phase process for the production of Linaclotide, preferably in the form of the acetate salt, which comprises the following steps:
a1) coupling a dipeptide Fragment B [7-8] of formula (II) Fmoc-Asn(Trt)-Pro-OH with a hexapeptide Fragment C [9-14] of formula (III) Fmoc-Ala-Cys[Ψ(Dmp,H)pro]-Thr(tBu)-Gly-Cys[Ψ(Dmp,H)pro]-Tyr(tBu)-OtBu to obtain the peptide [7-14];
a2) coupling the peptide [7-14] with an amino acid derivative of formula (IV) Fmoc-Cys(Trt)-OH in position 6 to obtain the peptide [6-14];
a3) coupling the peptide [6-14] with an amino acid derivative of formula (V) Fmoc-Cys(SO₃Na)-ONa in position 5 to obtain the peptide [5-14];
a4) coupling the peptide [5-14] with a tetrapeptide Fragment A [1-4] of formula (VI) Fmoc-Cys(SO₃Na)-Cys(SO₃Na)-Glu(tBu)-Tyr(tBu)-OH to obtain the linear protected peptide of formula (VII) Fmoc-Cys(SO₃Na)-Cys(SO₃Na)-Glu(tBu)-Tyr(tBu)-Cys(SO₃Na)-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu;
b) deprotecting compound (VII) by reaction with a secondary base and trifluoroacetic acid to obtain the intermediate of formula (VIII) H-Cys(SO₃H)-Cys(SO₃H)-Glu-Tyr-Cys(SO₃H)-Cys-Asn-Pro-Ala-Cys-Thr-Gly-Cys-Tyr-OH TFA salt;
c) carrying out a non-oxidative cyclization on intermediate (VIII) to obtain crude Linaclotide;
d) optionally, purifying the crude Linaclotide to obtain purified Linaclotide;
e) optionally, salifying the purified Linaclotide with acetic acid to form the corresponding acetate salt.

In a preferred embodiment of the process according to the invention, in step b) the secondary base is a secondary amine, preferably DEA, piperidine, piperazine or morpholine, and trifluoroacetic acid is at least 80% by volume in water.

In a preferred embodiment of the process according to the invention, step c) is performed by intramolecular nucleophilic substitution.

Preferably, step c) is performed in an hydroalcoholic buffer at pH comprised between 7 and 8.

In a further preferred embodiment, in step c) the crude Linaclotide is obtained in aqueous solution.

In a preferred embodiment of the process according to the invention, the coupling between (II) and (III) and/or the coupling between [7-14] and (IV) and/or the coupling between [6-14] and (V) and/or the coupling between [5-14] and (VI) is performed in the presence of ethyl-ciano-(hydroxyimino)-acetate also called Oxyma or its derivatives, such as PyOxim or TOTU, or 1,3-dimethylbarbituric acid derivatives, such as Oxyma-B.

In a further preferred embodiment of the process according to the invention, the coupling between (II) and (III) and/or the coupling between [7-14] and (IV) and/or the coupling between [6-14] and (V) and/or the coupling between [5-14] and (VI) is performed in organic polar solvents, preferably selected from DMF, NMP, ACN.

In a further preferred embodiment of the process according to the invention, the coupling between (II) and (III) and/or the coupling between [7-14] and (IV) and/or the coupling between [6-14] and (V) and/or the coupling between [5-14] and (VI) is performed in a temperature range between -10°C and 35°C, preferably between 0°C and 25°C.

In a preferred embodiment, the purification step d) is performed through Reverse Phase High Performance Sample Displacement (RP-HPSD), Reverse Phase High Performance Liquid Chromatograpy (RP-HPLC) or combination thereof.

In a preferred embodiment of the purification step d), the eluent phase consists of an aqueous solution, a polar organic solvent or a mixture thereof, preferably the polar organic solvent is selected from trifluoroacetic acid, acetic acid, acetonitrile and a mixture thereof, optionally with the addition of a buffer, more preferably a phosphoric buffer.

In a preferred embodiment, the purified Linaclotide is in solution, preferably said solution comprises water, acetic acid and acetonitrile.

Preferably, the process according to the invention allows to obtain amorphous Linaclotide having a chromatographic purity equal to or higher than 99.9%.

The process according to the invention may further comprise an isolation step f) by lyophilization starting from a hydroalcoholic solution or from an aqueous suspension.

Preferably, the hydroalcoholic solution contains tert-butanol, water, acetic acid or mixture thereof.

Preferably, the aqueous suspension is obtained by evaporation of the purified Linaclotide solution, more preferably the aqueous suspension contains acetic acid.

A further object of the present invention is amorphous Linaclotide or its acetate salt having a chromatographic purity equal to or higher than 99.9% and an amount of any of the following impurities lower than 100 ppm, preferably lower than 50 ppm (LOQ), more preferably lower than 40 ppm (LOD):
- IMD-Linaclotide (Impurity 1);
- Cys-1-α-ketone (Impurity 2).

Preferably, amorphous High-Purity Linaclotide or its acetate salt has a content of multimers equal to or lower than 0.1% area%.

A further object of the present invention is a method for detect (LOD 40 ppm) and quantify (LOQ 50 ppm) the impurity content of IMD-Linaclotide and Cys-1-α-ketone in the product amorphous High-Purity Linaclotide or its acetate salt, comprising the elution of the product through an Ion-Pairing Chromatography (IPC) column, having a silica stationary phase containing alkyl chains, and an eluent phase consisting of an aqueous solution, a polar organic solvent, or a mixture thereof, optionally with the addition of a buffer, preferably a phosphoric buffer.

In a preferred embodiment of the method according to the invention, said alkyl chains are of octadecyl, octyl, or butyl (C18, C8 or C4) type, preferably C18.

Preferably, said polar organic solvent is selected from tetrahydrofuran, dioxane, dicloromethane, methanol, ethanol, n-propanol, isopropanol, butanol, pentane, hexane, toluene, trifluoroacetic acid, acetonitrile, or a mixture thereof; more preferably trifluoroacetic acid, acetonitrile, or a mixture thereof.

In a further preferred embodiment, the method according to the invention is characterized in that it is performed according to the following operating conditions:
- Stationary phase: support silica particles containing C18 alkyl chains;
   Eluant A: phosphoric buffer pH 6.2
   Eluant B: ACN
   wherein the following gradient elution is applied:
      % B: 7-7(2');7-15(19');15-60(25').

The eluent phase of the method according to the invention contains an ion-pairing reagent, preferably heptanesulfonated salt.

### 1. CHEMICAL SYNTHESIS OF CRUDE LINACLOTIDE

In the manufacturing process which is an object of the present invention, crude Linaclotide is the first key intermediate, which is synthesized by a Liquid Phase Peptide Synthesis (LPPS) procedure, consisting of
1. a first phase of preparation of the linear protected peptide,
2. a second phase of deprotection to yield the linear advanced intermediate and
3. a final phase of cyclization with formation of the disulfide bridges that yields crude Linaclotide, according to the Scheme 1 reported below:

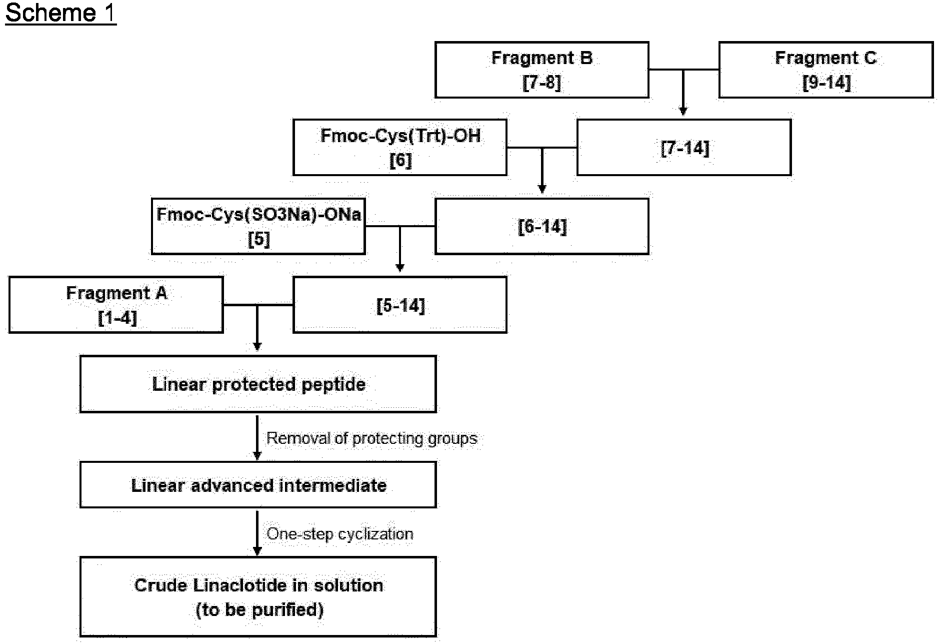

One of the key points of the synthesis of a complex peptide with multidisulfide bridges such as Linaclotide is to drive the cyclization step in the right way, setting the conditions to obtain the correct regioisomer according to the desired disulfide mapping, avoiding the formation of misfolding impurities as well as the formation of multimers.

To reach this target, a non oxidative cyclization method has been developed where an intramolecular nucleophilic substitution in an hydroalcoholic buffer at pH comprised between 7-8 has been carried out involving three nucleophilic moieties (thiols group of free Cys) and three leaving groups (sulfonated groups on the remaining Cys).

A set of trials have been preliminarily executed with the aim of selecting the best locations for the three sulfonated Cys groups along the backbone; all the possible combinations of the linear advanced peptide have been chemically prepared and submitted to cyclization.

The best results in terms of chromatographic purity and assay of the desired Linaclotide final product have been obtained for the trial where the sulfonated groups were positioned on the Cys in position 1, 2 and 5, whereas all the other combinations have led to worst results, and in some case, even to a chromatographic complex pattern of regioisomers without a main peak.

Therefore surprisingly, the intramolecular nucleophilic substitution is not only an advantageous synthetic method for the formation of one disulfide bridge, but it is particularly efficient for the formation of more than one disulfide bridge (e.g. for the manufacturing of complex multidisulfide bridges peptide such as Linaclotide).

Another key point in the synthesis of a complex multidisulfide bridges peptide such as Linaclotide is the choice of the protecting groups on the remaining Cys that need to cyclize with the above described sulfonated Cys (in the specific invention in position 6,10 and 13).

A set of trials have been preliminarly executed and the best results in terms of the final chromatographic purity, orthogonality, side-reactions and ease of handling have been obtained in the case of functionalization of the Cys in position 6 by Trt and of the Cys in position 10 and 13 by Cys-pseudoprolines.

The Cys-pseudoprolines represent a masked form of cysteine, having a five membered tiochetalic hindered ring with low tendency to racemization during coupling activation; the introduction of this moiety along the backbone has the additional advantage to guarantee higher solubility to the growing peptide as well to reduce the aggregation effects, leading to an improvement for the work-up.

The protection of the thiol groups of the Cys as pseudoprolines is a recent discovery and the relative uses in peptidic synthesis are still pioneristic, as of today, the available literature being related only to SPPS applications.

For this reason, the use of Cys-pseudoprolines in a LPPS manufacturing process as the one described hereby, represents an innovative application, considering also that the object of the synthesis is a complex Cys-rich peptide with multidisulfide bridges such as Linaclotide.

Once defined the positioning of the sulfonated groups along the linear protected peptide as well as the typology and the positioning of the protecting groups of the remaining Cys (which need to be deprotected before the cyclization step), a totally Liquid Phase Peptide Synthesis (LPPS) has been designed.

As preliminary synthetic steps to prepare the linear protected peptide, the following three fragments have to be synthesized, namely:
- Fragment A (VI), tetrapeptide containing the aminoacids of positions 1→4, where the Cys in position 1 and 2 are derivatized as sulfonated sodium salt

   **Fmoc-Cys(SO₃Na)-Cys(SO₃Na)-Glu(tBu)-Tyr(tBu)-OH** **(VI)**
- Fragment B (II), dipeptide containing the aminoacids of positions 7→8

   **Fmoc-Asn(Trt)-Pro-OH** **(II)**
- Fragment C (III), hexapeptide containing the aminoacids of positions 9→14, where the Cys in position 10 and 13 are protected as Cys-pseudoprolines (in symbol Cys[Ψ(Dmp,H)pro])

   **Fmoc-Ala-Cys[Ψ(Dmp,H)pro]-Thr(tBu)-Gly-Cys[Ψ(Dmp,H)pro]-Tyr(tBu)-OtBu** **(III)**

Each of the three fragments is characterized by a HPLC Purity not lower than 90%.

For the following step of preparation of the linear protected peptide, a convergent assembling of the Fragment A (VI), Fragment B (II), Fragment C (III) and the two aminoacid derivatives Fmoc-Cys(Trt)-OH (IV) in position 6 and Fmoc-Cys(SO₃Na)-ONa (V) in position 5 is carried out, leading to the obtainment of the following 14-mer linear protected peptide (VII):

**Fmoc-Cys(SO₃Na)-Cys(SO₃Na)-Glu(tBu)-Tyr(tBu)-Cys(SO₃Na)-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu** **(VII)**

The assembling of Fragment A (VI), Fragment B (II), Fragment C (III) and the two aminoacids derivatives Fmoc-Cys(Trt)-OH (IV) in position 6 and Fmoc-Cys(SO₃Na)-ONa (V) in position 5, to obtain linear protected peptide is carried out by the repetition of a simple, industrially applicable and robust synthetic protocol summarized in the below reported Scheme 2:

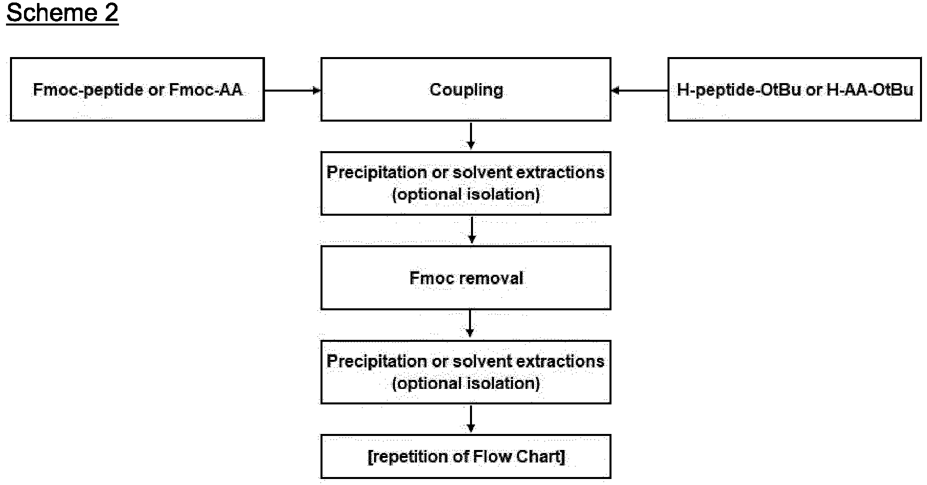

The linear protected peptide is characterized by an HPLC Purity not less than 65%. In the following later step of preparation of the linear advanced intermediate, the removal of the protecting groups is carried on in 2 different steps, namely:
- Fmoc removal mediated by a secondary base
- Acid-labile protecting groups removal (such as tBu esters, Trt and pseudoprolines) mediated by TFA of at least 80% by volume in water
that finally result in the obtainment of the linear advanced intermediate (VIII):

**H-Cys(SO₃H)-Cys(SO₃H)-Glu-Tyr-Cys(SO₃H)-Cys-Asn-Pro-Ala-Cys-Thr-Gly-Cys-Tyr-OH TFA salt** **(VIII)**

The linear advanced intermediate is characterized by an HPLC purity of at least 55%.

In the final step to produce crude Linaclotide in solution, the cyclization of the linear advanced intermediate is carried out by a manufacturing method having the following characteristics:
a. it is a non-oxidative method: the folding is obtained by intramolecular nucleophilic substitution-based reactions where the free thiol groups of the Cys in position 6, 10 and 13 play the role of the nucleophilic moieties and the sulfonated groups of Cys in position 1, 2 and 5 are the leaving groups.
b. it is a semi-regioselective method, where the number of the obtainable regioisomers is lower than the statistically possible combinations: this goal is achieved by a unique step reaction which is different and more advantageous than the sequential cyclizations currently described in the existing semi-regioselective methods and allows to obtain the crude Linaclotide in solution:

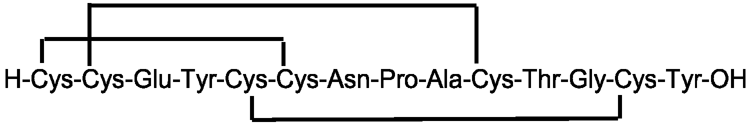

Crude Linaclotide in solution is typically obtained with an HPLC Purity of 45-55%, which is particularly surprising and advantageous because such degree of purity is reached without any intermediate purification, allowing saving of time, lower costs and increase of productivity, as compared to existing methods.

### 2. PURIFICATION AND ISOLATION OF HIGH-PURITY LINACLOTIDE

Crude Linaclotide is further processed by the application of a combination of 2 purification techniques: Reverse Phase High Performance Sample Displacement (RP-HPSD) and Reverse Phase High Performance Liquid Chromatographic (RP-HPLC) and is finally isolated by application of any of 2 different manufacturing options starting from Linaclotide purified solution (which is an ACN-water solution containing AcOH):

### MANUFACTURING OPTION 1:

solvent switch from ACN to t-BuOH and lyophilization from t-BuOH-water (containing AcOH) solution

### MANUFACTURING OPTION 2:

concentration and lyophilization from aqueous (containing AcOH) suspension according to the Flow Chart reported in Scheme 3:

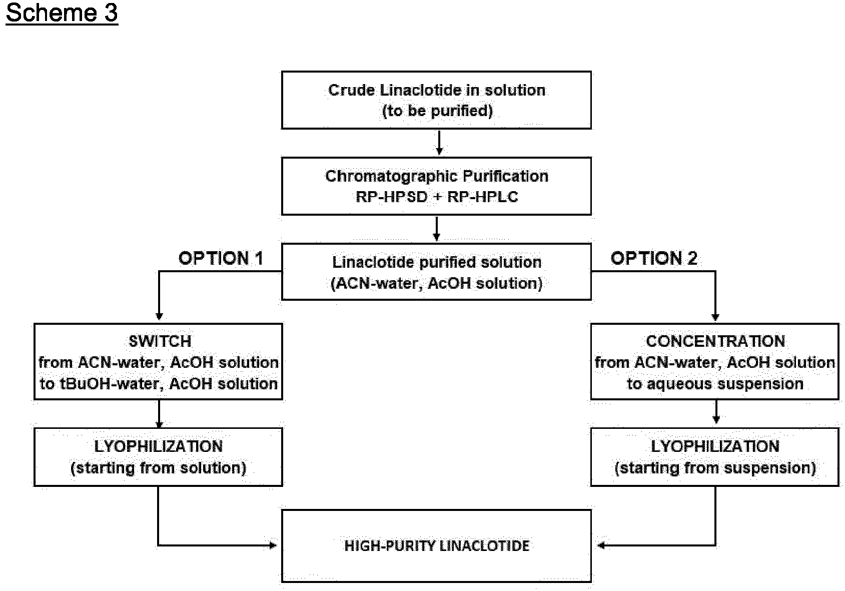

Going into details, the step of chromatographic purification is carried out by application on the crude Linaclotide solution of a combination of 2 different techniques: RP-HPSD + RP-HPLC.

The RP-HPSD method works based on the concept that the sample molecules bind to the column and separate from one another depending on their affinities with the stationary phase. The technique foresees an overloading of the sample charge on the column and guarantees the separation of Linaclotide from its impurities by a mild slope gradient with a low % of organic solvent.

The overall chromatographic purification process can be represented in the Scheme 4 reported below:

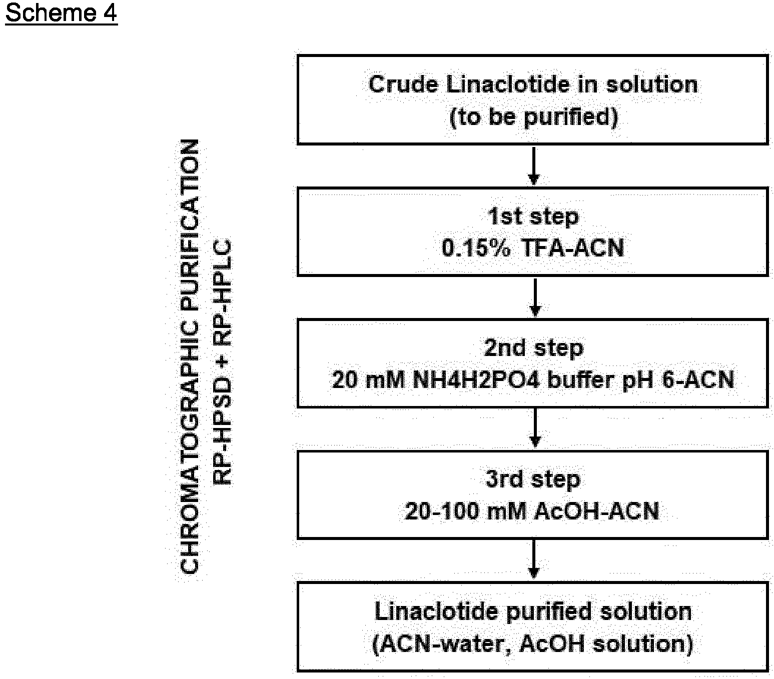

At the end of the purification process, purified Linaclotide in solution is obtained having a chromatographic purity 99.9% minimum, starting from crude Linaclotide in solution (HPLC Purity about 45-55%). The residual amount of both IMD-Linaclotide and Cys-1-α-ketone-Linaclotide impurities does not exceed 100 ppm, each one being typically lower than 50 ppm (LOQ) and more typically lower than 40 ppm (LOD). Once the purified Linaclotide solution is obtained, the final amorphous High-Purity Linaclotide can be isolated by application of one of the following alternative manufacturing options:

### MANUFACTURING OPTION 1:

Linaclotide purified solution is loaded on a RP-column and a switch ACN→tBuOH is performed by eluting the column with a phase composed of a 1 : 1 mixture of Phase 1 (water-AcOH 100 mM) and Phase 2 (t-BuOH).

Then the t-BuOH-water (containing AcOH) solution is lyophilized to obtain amorphous High-Purity Linaclotide powder.

### MANUFACTURING OPTION 2:

Linaclotide purified solution is concentrated under vacuum to remove ACN.

Then the obtained aqueous (AcOH) suspension is transferred into trays to be lyophilized to obtain amorphous High-Purity Linaclotide powder.

### 3. API CHARACTERIZATION

Amorphous High-Purity Linaclotide is characterized as follows:

### A. Purity, Assay and evaluation of impurities IMD-Linaclotide and Cys-1-α-ketone-Linaclotide

### by Ion-Pairing Chromatography (IPC)

The analytical method is based on the principles of the Ion-Pairing Chromatography (IPC): this technique allows the separation of ionic analytes using a mobile phase which contains a specific modifier consisting of lipophilic ions of opposite charge with respect to the analytes. The lipophilic ions of the mobile phase interact with the analytes balancing their ionic charges thus allowing separation on the stationary phase.

Optimization of the analytical variables allows also the separation of complex sample mixtures containing both ionic/ionizable species and neutral analytes.

### Analytical method description

Column: Gemini-NX, 5µm, 4.6 ×250 mm
Eluant A: 20 mM (NH₄)₃PO₄ buffer pH 6.2 +
5 mM sodium 1- heptanesulfonate monohydrate (ion-pairing reagent)
Eluant B: ACN
Gradient % B: 7-7(2');7-15(19');15-60(25')
Flow: 1.0 ml/min; T: 30°C, UV: 220 nm
   IMD-Linaclotide RRT 0.84-0.85
   Cys-1-α-ketone-Linaclotide RRT 0.91-0.92
   Limit of detection (LOD): 40 ppm
   Limit of quantification (LOQ): 50 ppm

### B. Multimers content

### by Gel Permetion Chromatography (GPC)

### Analytical method description

Column: TSK Gel G2000SWXL, 5µm, 7.8 ×300 mm
Eluant: 70% ACN + 0.02% TFA
Gradient: isocratic (40')
Flow: 0.6 ml/min; T: 30°C, UV: 215 nm
   Multimers (Sum of the peaks) RRT ≤ 0.81-0.82

### C. Physical form

The physical form of the amorphous High-Purity Linaclotide is evaluated by the following analyses:
- PXRD (Powder X-Ray Diffraction) for the assessment of the crystalline structure
- DSC (Differential Scanning Calorimetry) for the evaluation of endo/exothermic events related to the presence of possible crystalline contaminants.

The following examples are intended to further illustrate the invention but not limiting it.

### EXAMPLE 1

### Synthesis of Fragment A [1-5]

### Fmoc-Cys(SO₃Na)-Cys(SO₃Na)-Glu(tBu)-Tyr(tBu)-OH [M.W. 1055.14, disodium salt]

### STEP I-Synthesis of H-Glu(tBu)-Tyr(tBu)-OAII TFA salt

1240 grams of Trt-Glu(tBu)-OH DCHA salt were dissolved into 8.7 L of iPrOAc and the organic solution was washed with an aqueous solution of KHSO₄, then concentrated and the residue dissolved in NMP (5.6 L).

H-Tyr(tBu)-OAll HCl salt (590 grams) was added and the coupling reaction carried out by PyOxim/NMM system

The reaction mixture was diluted with iPrOAc and the peptide rich organic solution was washed with an aqueous solution of NaHCO₃ and NaCl, then concentrated and the residue dissolved in DCM (6.1 L).

The acidic deprotection was carried out by a mix TFA-TIS.

The reaction solution was concentrated and precipitated by a mix n-heptane-DIPE, filtered, washed and dried.

H-Glu(tBu)-Tyr(tBu)-OAll TFA salt was obtained with Purity HPLC 99.8% and Yield 89%.

### STEP II-Synthesis of Fmoc-Cys(SO₃Na)-Cys(SO₃Na)-Glu(tBu)-Tyr(tBu)-OH

952 grams of H-Glu(tBu)-Tyr(tBu)-OAII TFA salt and 810 grams of Fmoc-Cys(SO₃Na)-ONa were dissolved in 4.8 L of DMF and the coupling reaction carried out by PyOxim/2,4, 6 collidine system.

The reaction mixture was diluted with iPrOAc and the peptide rich organic solution was washed with an aqueous solution of KHSO₄, NaHCO₃ and NaCl, then concentrated and the residue dissolved in iPrOAc. The basic deprotection was carried out by DEA.

The reaction mixture was diluted with iPrOAc and the peptide rich organic solution was washed with an aqueous solution of NaHCO₃, KHSO₄ and NaCl, then concentrated, and the residue dissolved in DMF (7.9 L).

Fmoc-Cys(SO₃Na)-ONa (695 grams) was added and the coupling reaction carried out by PyOxim/2,4,6 collidine system.

The reaction mixture was diluted with DCM and the peptide rich organic solution was washed with an aqueous solution of KHSO₄, NaHCO₃ and NaCl, then concentrated and the residue dissolved in DCM (19.7 L).

The allylester removal was carried out by PhSiH₃ and Pd(PPh₃)₄.

The reaction mixture was quenched with addition of an aqueous solution of NaCl, the phases were separated and to the organic phase an aqueous solution of NaHCO₃ was added. The peptide was extracted from the aqueous phase by further addition of DCM.

The organic phase was concentrated and precipitated by addition of MTBE, filtered, washed and dried.

Fmoc-Cys(SO₃Na)-Cys(SO₃Na)-Glu(tBu)-Tyr(tBu)-OH was obtained with Purity HPLC 94.3% and Yield 65%.

### EXAMPLE 2

### Synthesis of Fragment B [7-8]

### Fmoc-Asn(Trt)-Pro-OH [M.W. 693.80]

177 grams of H-Pro-OBzl HCl salt was suspended into 1.8 L of DMF. Fmoc-Asn(Trt)-OH (350 grams) was added and the coupling reaction carried out by PyOxim/2,4,6 collidine system.

The reaction mixture was precipitated by addition of an aqueous solution of NaHCO₃, filtered, washed and dried.

The dried product was hydrogenated to remove benzylester protection using IPA (17.0 L) and water (1.0 L) as reaction solvents and Pd/C 5% (50% wet) (120 grams) as catalyst. The reaction was kept under H₂ for not less than 3h50'. The suspension was filtered and the catalyst cake was washed by a mix IPA-water and the collected solutions were precipitated by charging an aqueous solution of KHSO₄. The suspension was filtered, washed and dried.

Fmoc-Asn(Trt)-OH was obtained with Purity HPLC 97.0% and Yield 79%.

### EXAMPLE 3

### Synthesis of Fragment C [9-14]

### Fmoc-Ala-Cys[Ψ(Dmp,H)pro]-Thr(tBu)-Gly-Cys[Ψ(Dmp,H)pro]-Tyr(tBu)-OtBu [M.W. 1303.60]

### STEP I-Synthesis of Fmoc-Gly-Cys[Ψ(Dmp,H)pro]-Tyr(tBu)-OtBu

950 grams of Fmoc-Gly-Cys[Ψ(Dmp,H)pro]-OH and 582 grams of H-Tyr(tBu)-OtBu HCl salt were dissolved into 4.8 L of NMP and the coupling reaction carried out by TOTU/NMM system.

The reaction mixture was precipitated by an aqueous solution of KHSO₄, filtered, washed and dried.

Fmoc-Gly-Cys[Ψ(Dmp,H)pro]-Tyr(tBu)-OtBu was obtained with Purity HPLC 98.8% and Step Yield 90%

### STEP II-Synthesis of H-Gly-Cys[Ψ(Dmp,H)pro]-Tyr(tBu)-OtBu

1277 grams of Fmoc-Gly-Cys[Ψ(Dmp,H)pro]-Tyr(tBu)-OtBu was dissolved into 5.7 L of ACN. The basic deprotection was carried out by DEA.

The reaction mixture was diluted with iPrOAc and the peptide rich organic solution was washed with an aqueous solution of KHSO₄ and NaCl, then concentrated and the residue dissolved into iPrOAc.

The solution was precipitated by addition of n-heptane, filtered, washed and dried. H-Gly-Cys[Ψ(Dmp,H)pro]-Tyr(tBu)-OtBu was obtained with Purity HPLC 95.1% and Yield 93%.

### STEP III-Synthesis of Fmoc-Thr(tBu)-Gly-Cys[Ψ(Dmp,H)pro]-Tyr(tBu)-OtBu

865 grams of H-Gly-Cys[Ψ(Dmp,H)pro]-OH and 572 grams of Fmoc-Thr(tBu)-OH were dissolved into 4.3 L of NMP and the coupling reaction carried out by PyOxim/NMM system.

The reaction mixture was diluted with IPA and precipitated by addition of an aqueous solution of NaHCO₃, filtered, washed and dried.

Fmoc-Thr(tBu)-Gly-Cys[Ψ(Dmp,H)pro]-Tyr(tBu)-OtBu was obtained with Purity HPLC 93.9% and Yield 87%

### STEP IV-Synthesis of Fmoc-Ala-Cys[Ψ(Dmp,H)pro]-Thr(tBu)-Gly-Cys[Ψ(Dmp,H)pro]-Tyr(tBu)-OtBu

1224 grams of Fmoc-Thr(tBu)-Gly-Cys[Ψ(Dmp,H)pro]-Tyr(tBu)-OtBu was dissolved into 6.1 L of iPrOAc. The basic deprotection was carried out by DEA.

The reaction mixture was diluted with iPrOAc and the peptide rich organic solution was washed with an aqueous solution of KHSO₄ and NaCl, then concentrated, and the residue dissolved into NMP (6.1 L).

Fmoc-Ala-Cys[Ψ(Dmp,H)pro]-OH (688 grams) was added and the coupling reaction was carried out by TOTU/NMM system.

The reaction mixture was diluted with IPA and precipitated by an aqueous solution of NaHCO₃, filtered and washed.

The product was then dried.

Fmoc-Ala-Cys[Ψ(Dmp,H)pro]-Thr(tBu)-Gly-Cys[Ψ(Dmp,H)pro]-Tyr(tBu)-OtBu was obtained with Purity HPLC 91.9% and Yield 93%

### EXAMPLE 4

### Synthesis of linear protected Linaclotide

### Fmoc-Cys(SO₃Na)-Cys(SO₃Na)-Glu(tBu)-Tyr(tBu)-Cys(SO₃Na)-Cys(Trt)-Asn(Trt)-Pro-Ala-Cys[Ψ(Dmp,H)prol-Thr(tBu)-Gly-Cys[Ψ(Dmp,H)prol-Tyr(tBu)-OtBu

### STEP I-Synthesis of H-Ala-Cys[Ψ(Dmp,H)pro]-Thr(tBu)-Gly-Cys[Ψ(Dmp,H)pro]-Tyr(tBu)-OtBu

1476 grams of Fragment C was dissolved into 6.6 L of ACN. The basic deprotection was carried out by DEA.

The reaction mixture was diluted with iPrOAc and the peptide rich organic solution was washed with an aqueous solution of KHSO₄ and NaCl, then concentrated and the residue dissolved into iPrOAc.

The solution was precipitated by addition of DIPE, filtered, washed and dried. H-Ala-Cys[Ψ(Dmp,H)pro]-Thr(tBu)-Gly-Cys[Ψ(Dmp,H)pro]-Tyr(tBu)-OtBu was obtained with Purity HPLC 90.3% and Yield 91%

### STEP II-Synthesis of Fmoc-Asn(Trt)-ProAla-Cys[Ψ(Dmp,H)pro]-Thr(tBu)-Gly-Cys[Ψ(Dmp,H)pro]-Tyr(tBu)-OtBu

1087 grams of H-Ala-Cys[Ψ(Dmp,H)pro]-Thr(tBu)-Gly-Cys[Ψ(Dmp,H)pro]-Tyr(tBu)-OtBu and 698 grams of Fragment B were dissolved into 5.4 L of ACN and the coupling reaction carried out by Oxyma-EDC*HCl/NMM system.

The reaction mixture was precipitated by the addition of an aqueous solution of NaHCO₃, filtered, washed and dried.

Fmoc-Asn(Trt)-Pro-Ala-Cys[Ψ(Dmp,H)pro]-Thr(tBu)-Gly-Cys[Ψ(Dmp,H)pro]-Tyr(tBu)-OtBu was obtained with Purity HPLC 87.3% and Yield 98%

### STEP III-Synthesis of H-Asn(Trt)-Pro-Ala-Cys[Ψ(Dmp,H)pro]-Thr(tBu)-Gly-Cys[Ψ(Dmp,H)pro]-Tyr(tBu)-OtBu

1727 grams of Fmoc-Asn(Trt)-Pro-Ala-Cys[Ψ(Dmp,H)pro]-Thr(tBu)-Gly-Cys[Ψ(Dmp,H)pro]-Tyr(tBu)-OtBu was dissolved into 8.6 L of iPrOAc. The basic deprotection was carried out by DEA.

The reaction mixture was diluted with iPrOAc and the peptide rich organic solution was washed with an aqueous solution of KHSO₄ and NaCl, then concentrated, and the residue dissolved into iPrOAc.

The solution was precipitated by addition of DIPE, filtered, washed.

The product was then dried.

H-Asn(Trt)-Pro-Ala-Cys[Ψ(Dmp,H)pro]-Thr(tBu)-Gly-Cys[Ψ(Dmp,H)pro]-Tyr(tBu)-OtBu was obtained with Purity HPLC 89.0% and Yield 89%

### STEP IV-Synthesis of Fmoc-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu

1339 grams of H-Asn(Trt)-Pro-Ala-Cys[Ψ(Dmp,H)pro]-Thr(tBu)-Gly-Cys[Ψ(Dmp,H)pro]-Tyr(tBu)-OtBu and 512 grams of Fmoc-Cys(Trt)-OH were dissolved into 6.7 L of DMF and the coupling reaction was carried out by TOTU/NMM system.

The reaction mixture was precipitated by an aqueous solution of NaHCO₃, filtered, washed dried.

Fmoc-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu was obtained with Purity HPLC 84.3% and Yield 95%

### STEP V-Synthesis of H-Cys(SO3Na)-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu

1733 grams of Fmoc-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu were dissolved into 8.7 L of iPrOAc. The basic deprotection was carried out by DEA.

The reaction mixture was diluted with iPrOAc and the peptide rich organic solution was washed firstly with an aqueous solution of KHSO₄, NaHCO₃ and NaCl, then concentrated, and the obtained residue was dissolved into DMF (8.7 L). Fmoc-Cys(SO₃Na)-ONa (404 grams) was added and the coupling reaction carried out by PyOxim/2,4,6 collidine system.

The reaction mixture was precipitated by an aqueous solution of NaHCO3, filtered, washed and dried.

The dried product was dissolved in 8.7 L of iPrOAc. The basic deprotection was carried out by DEA.

The reaction mixture was diluted with iPrOAc and the peptide rich organic solution was washed with an aqueous solution of KHSO₄ and NaCl, then concentrated, and the obtained residue was dissolved into iPrOAc.

The obtained solution was precipitated by addition of DIPE, filtered and washed. The product was then dried.

H-Cys(SO₃Na)-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu was obtained with Purity HPLC 82.9% and Yield 83%

### STEP VI-Synthesis of linear protected peptide

*Fmoc-Cys(SO₃Na)-Cys(SO₃Na)-Glu(tBu)-Tyr(tBu)-Cys(SO₃Na)-Cys(Trt)Asn(Trt)-Pro-Ala-Cys[Ψ(Dmp,H)pro]-Thr(tBu)-Gly-Cys[Ψ(Dmp,H)pro]-Tyr(tBu)-OtBu*

1400 grams of H-Cys(SO₃Na)-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu and 850 grams of Fragment A were dissolved into 7.0 L of ACN and the coupling reaction carried out by Oxyma/B-EDC*HCl/2,4,6 collidine system.

The reaction mixture was precipitated by an aqueous solution of NaHCO₃, filtered, washed and dried.

Linear protected peptide was obtained with Purity HPLC 66.7% and quantitative Yield.

### EXAMPLE 5

### Synthesis of linear advanced intermediate

### H-Cys(SO₃H)-Cys(SO₃H)-Glu-Tyr-Cys(SO₃H)-Cys-Asn-Pro-Ala-Cys-Thr-Gly-Cys-Tyr-OH TFA salt [M.W. 1772.98, free base]

### STEP I-Synthesis of H-Cys(SO₃Na)-Cys(SO₃Na)-Glu(tBu)-Tyr(tBu)-Cys(SO₃Na)-Cys(Trt)-Asn(Trt)-Pro-Ala-Cys[Ψ(Dmp,H)pro]-Thr(tBu)-Gly-Cys(Ψ(Dmp,H)pro]-Tyr(tBu)-OtBu

2126 grams of linear protected peptide was dissolved into 10.6 L of ACN. The basic deprotection was carried out by DEA.

The reaction mixture was diluted with iPrOAc and the peptide rich organic solution was washed with an aqueous solution of KHSO₄ and NaCl, then concentrated, and the obtained residue was dissolved into iPrOAc.

The solution was precipitated by addition of n-heptane, filtered, washed and dried. H-Cys(SO₃Na)-Cys(SO₃Na)-Glu(tBu)-Tyr(tBu)-Cys(SO₃Na)-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu was obtained with Purity HPLC 61.7% and Yield 89%

### STEP II-Synthesis of linear advanced intermediate

*H-Cys(SO₃H)-Cys(SO₃H)-Glu-Tyr-Cys(SO₃H)-Cys-Asn-Pro-Ala-Cys-Thr-Gly-Cys-Tyr-OH TFA salt*

450 grams of H-Cys(SO₃Na)-Cys(SO₃Na)-Glu(tBu)-Tyr(tBu)-Cys(SO₃Na)-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu were treated with a solution of TFA-water-TIS (20.3 L-2.3 L-0.5 L) The reaction mixture was then precipitated by addition of MTBE filtered, washed and dried.

Linear advanced intermediate was obtained with Purity HPLC 57.9% and Yield 92%.

### EXAMPLE 6

### Synthesis of crude Linaclotide in solution

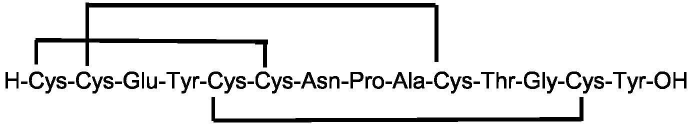

[M.W.1526.74]

90 grams of linear advanced intermediate (estimated 49 grams of peptide 100%) was charged in a mix phosphate buffer pH=7.4 and IPA and the reaction mixture was kept stirring for 16-32 hours.

The mixture was concentrated to remove IPA and then filtered.

Crude Linaclotide in solution (30 grams peptide content) was obtained with Purity HPLC 53.8% and cyclization Yield 72%.

### EXAMPLE 7

### Purification of crude Linaclotide in solution

Crude Linaclotide in solution (75.4 grams Linaclotide content) was loaded onto C18 column (100 mm diameter x 350 mm max height) and purified by applying 3 different and consecutive chromatographic steps

### -1st step

Gradient: from 0% to 100% of Eluent B in 180 minutes
Eluent A: 0.15% TFA
Eluent B: Eluent A-ACN 1-1

### -2nd step

Gradient: from 0% to 100% of Eluent B in 180 minutes
Eluent A: 20 mM NH₄H₂PO₄ buffer pH 6
Eluent B: Eluent A-ACN 1-1

### -3rd step

Gradient: from 0% to 100% of Eluent B in 180 minutes
Eluent A: 20-100 mM AcOH
Eluent B: Eluent A-ACN 1-1
Finally, 41.7 grams of Linaclotide purified in solution were obtained starting from 75.4 grams; chromatographic purity 99.97% and Yield (3 steps) 55%.

### EXAMPLE 8

### HIGH-PURITY LINACLOTIDE ISOLATION FROM tBuOH-WATER SOLUTION (OPTION 1)

Linaclotide purified solution (41.7 grams, Linaclotide concentration 4.2 g/L) was processed as follows:
- switch from ACN to tBuOH was carried out by charging the above solution on the C18 column of the previous example and eluting with an AcOH (100 mM)-tBuOH 1-1 solution, to obtain a final solution of Linaclotide in water (AcOH) and tBuOH;
- the above solution was lyophilized according to the following cycle:
   Freezing of the matrix at -50°C, under ordinary pressure;
   Primary Lyophilization stage at -20°C, under vacuum;
   Secondary Lyophilization stage at +20°C up to dryness of the lyophilized product, under vacuum;
   to obtain 46.4 grams of amorphous High-Purity Linaclotide powder corresponding to 40.9 grams Linaclotide 100% (Assay 88.1%).

The obtained product has the following analytical attributes:
- Chromatographic Purity (IPC) 99.94% (FIG-1),
- IMD-Linaclotide LT 40 ppm (LT LOD) (FIG-4),
- Cys-1-α-ketone-Linaclotide LT 40 ppm (LT LOD) (FIG-4)
- Multimers content 0.07% (FIG-6)
- Isolation Yield 98%.

The obtained product was submitted to PXRD and DSC analyses which confirm that the powder is totally amorphous (FIG-8, FIG-9).

### EXAMPLE 9

### HIGH-PURITY LINACLOTIDE ISOLATION FROM AQUEOUS SUSPENSION (OPTION 2)

Linaclotide purified solution (42.9 grams Linaclotide content, concentration 4.3 g/L) was processed as follows:
- concentration under vacuum was carried out to remove ACN and to obtain an aqueous suspension;
   the above suspension was lyophilized, according to the following cycle:
   Freezing of the matrix at -50°C, under ordinary pressure;
   Primary Lyophilization stage at -20°C, under vacuum;
   Secondary Lyophilization stage at +20°C up to dryness of the lyophilized product, under vacuum;
to obtain 40.9 grams of amorphous High-Purity Linaclotide powder corresponding to 39.1 grams Linaclotide 100% (Assay 95.7%).

The obtained product has the following analytical attributes:
- Chromatographic Purity (IPC) 99.91% (FIG-2),
- IMD-Linaclotide LT 40 ppm (LT LOD) (FIG-5),
- Cys-1-α-ketone-Linaclotide LT 40 ppm (LT LOD) (FIG-5),
- Multimers content 0.06% (FIG-7)
- Isolation Yield 91%.

The obtained product was submitted to PXRD and DSC analysis and the powder was confirmed to be a totally amorphous solid (FIG-10, FIG-11).

## Claims

1. A liquid-phase process for the production of Linaclotide, preferably in the form of the acetate salt, which comprises the following steps:
a1) coupling a dipeptide Fragment B [7-8] of formula (II) Fmoc-Asn(Trt)-Pro-OH with a hexapeptide Fragment C [9-14] of formula (III) Fmoc-Ala-Cys[Ψ(Dmp,H)pro]-Thr(tBu)-Gly-Cys[Ψ(Dmp,H)pro]-Tyr(tBu)-OtBu to obtain the peptide [7-14];
a2) coupling the peptide [7-14] with an amino acid derivative of formula (IV) Fmoc-Cys(Trt)-OH in position 6 to obtain the peptide [6-14];
a3) coupling the peptide [6-14] with an amino acid derivative of formula (V) Fmoc-Cys(SO₃Na)-ONa in position 5 to obtain the peptide [5-14];
a4) coupling the peptide [5-14] with a tetrapeptide Fragment A [1-4] of formula (VI) Fmoc-Cys(SO₃Na)-Cys(SO₃Na)-Glu(tBu)-Tyr(tBu)-OH to obtain the protected peptide of formula (VII) Fmoc-Cys(SO₃Na)-Cys(SO₃Na)-Glu(tBu)-Tyr(tBu)-Cys(SO₃Na)-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu;
b) deprotecting compound (VII) by reaction with a secondary base and trifluoro acetic acid to obtain the intermediate of formula (VIII) H-Cys(SO₃H)-Cys(SO₃H)-Glu-Tyr-Cys(SO₃H)-Cys-Asn-Pro-Ala-Cys-Thr-Gly-Cys-Tyr-OH TFA salt;
c) carrying out a non-oxidative cyclization on intermediate (VIII) to obtain crude Linaclotide;
d) optionally, purifying the crude Linaclotide to obtain purified Linaclotide;
e) optionally, salifying the purified Linaclotide with acetic acid to form the corresponding acetate salt.

2. A process according to claim 1, wherein in step b) the secondary base is a secondary amine, preferably DEA, piperidine, piperazine or morpholine, and trifluoro acetic acid is at least 80% by volume in water.

3. A process according to claim 1 or 2, wherein step c) is performed by intramolecular nucleophilic substitution.

4. A process according to any one of the preceding claims, wherein step c) is performed in an hydroalcoholic buffer at pH comprised between 7 and 8.

5. A process according to any one of the preceding claims, wherein in step c) the crude Linaclotide is obtained in aqueous solution.

6. A process according to any one of the preceding claims, wherein the coupling between (II) and (III) and/or the coupling between [7-14] and (IV) and/or the coupling between [6-14] and (V) and/or the coupling between [5-14] and (VI) is performed in the presence of ethyl-cyano-(hydroxyimino)-acetate or its derivatives, or 1,3-dimethylbarbituric acid derivatives.

7. A process according to any one of the preceding claims, wherein the coupling between (II) and (III) and/or the coupling between [7-14] and (IV) and/or the coupling between [6-14] and (V) and/or the coupling between [5-14] and (VI) is performed in organic polar solvents, preferably selected from DMF, NMP, ACN.

8. A process according to any one of the preceding claims, wherein the coupling between (II) and (III) and/or the coupling between [7-14] and (IV) and/or the coupling between [6-14] and (V) and/or the coupling between [5-14] and (VI) is performed in a temperature range between -10°C and 35°C, preferably between 0°C and 25°C.

9. A process according to any one of the preceding claims, wherein the purification step d) is performed through Reverse Phase High Performance Sample Displacement (RP-HPSD), Reverse Phase High Performance Liquid Chromatograpy (RP-HPLC) or combination thereof.

10. A process according to claim 9, wherein the eluent phase consists of an aqueous solution, a polar organic solvent or a mixture thereof, preferably the polar organic solvent is selected from trifluoroacetic acid, acetic acid, acetonitrile and a mixture thereof, optionally with the addition of a buffer, preferably a phosphoric buffer.

11. A process according to claim 9 or 10, wherein the purified Linaclotide is in solution, preferably said solution comprises water, acetic acid and acetonitrile.

12. A process according to any one of claims 9 to 11, wherein Linaclotide has an HPLC purity equal to or higher than 99.9% and a content of IMD-Linaclotide and Cys-1-α-ketone-Linaclotide impurities each one lower than 100 ppm, preferably lower than 50 ppm, more preferably lower than 40 ppm.

13. A process according to any one of the preceding claims, further comprising an isolation step f) by lyophilization starting from a hydroalcoholic solution or from an aqueous suspension.

14. A process according to claim 13, wherein the hydroalcoholic solution contains tert-butanol, water, acetic acid or mixture thereof.

15. A process according to claim 13, wherein the aqueous suspension is obtained by evaporation of the purified Linaclotide solution, preferably the aqueous suspension contains acetic acid.

16. A method for the detection and the quantification of IMD-Linaclotide and Cys-1-α-ketone in the amorphous High-Purity Linaclotide or its acetate salt, comprising the elution of the product through a Ion-Pairing Chromatography (IPC) column, having a silica stationary phase containing alkyl chains, and an eluent phase consisting of an aqueous solution, a polar organic solvent, or a mixture thereof, optionally with the addition of a buffer, preferably a phosphoric buffer; wherein the eluent phase contains an ion-pairing reagent, preferably heptanesulfonate salt.

17. The method according to claim 16, **characterized in that** said alkyl chains are of octadecyl, octyl, or butyl (C18, C8 or C4) type, preferably C18.

18. The method according to claim 16 or 17, **characterized in that** said polar organic solvent is selected from tetrahydrofuran, dioxane, dichloromethane, methanol, ethanol, n-propanol, isopropanol, butanol, pentane, hexane, toluene, trifluoroacetic acid, acetonitrile, or a mixture thereof, preferably trifluoroacetic acid, acetonitrile, or a mixture thereof.

19. The method according to any one of claims 16 to 18, **characterized in that** it is performed according to the following operating conditions:
- Stationary phase: support silica particles containing C18 alkyl chains;
Eluant A: phosphoric buffer pH 6.2 + sodium 1-heptanesulfonate (ion pairing agent)
Eluant B: ACN
wherein the following gradient elution is applied:
% B: 7-7(2');7-15(19');15-60(25'),
and the following conditions are implemented:
Flow: 1.0 ml/min; Column T: 30°C, UV 220 nm.

20. Amorphous Linaclotide or its acetate salt having chromatographic purity equal to or higher than 99.9% and an amount of any of the following impurities lower than 100 ppm, preferably lower than 50 ppm, more preferably lower than 40 ppm:
- IMD-Linaclotide (Impurity 1);
- Cys-1-α-ketone (Impurity 2);
wherein the detection and the quantification of IMD-Linaclotide and Cys-1-α-ketone are performed according to the method as defined in any one of claims 16 to 19.

21. Amorphous High-Purity Linaclotide or its acetate salt according to claim 20, having a content of multimers equal to or lower than 0.1% area%.

## Patentansprüche

1. Flüssigphasenprozess zur Herstellung von Linaclotid₁ vorzugsweise in Form des Acetatsalzes, der die folgenden Schritte umfasst:
a1) Koppeln eines Dipeptidfragments B [7-8] der Formel (II) Fmoc-Asn(Trt)-Pro-OH mit einem Hexapeptidfragment C [9-14] der Formel (III) Fmoc-Ala-Cys[Ψ(Dmp,H)pro]-Thr(tBu)-Gly-Cys[Ψ(Dmp,H)pro]-Tyr(tBu)-OtBu, um das Peptid [7-14] zu erhalten;
a2) Koppeln des Peptids [7-14] mit einem Aminosäurederivat der Formel (IV) Fmoc-Cys(Trt)-OH in Position 6, um das Peptid [6-14] zu erhalten;
a3) Koppeln des Peptids [6-14] mit einem Aminosäurederivat der Formel (V) Fmoc-Cys(SO₃Na)-ONa in Position 5, um das Peptid [5-14] zu erhalten;
a4) Koppeln des Peptids [5-14] mit einem Tetrapeptidfragment A [1-4] der Formel (VI) Fmoc-Cys(SO₃Na)-Cys(SO₃Na)-Glu(tBu)-Tyr(tBu)- OH, um das geschützte Peptid der Formel (VII) Fmoc-Cys(SO₃Na)-Cys(SO₃Naa)-Glu(tBu)-Tyr(tBu)-Cys(SO₃Na)-Cys(Trt)-Asn(Trt)-Pro-Ala-Cys[Ψ(Dmp, H)pro]-Thr(tBu)-Gly-Cys[Ψ(Dmp,H)pro]-Tyr(tBu)-OtBu zu erhalten;
b) Entschützen der Verbindung (VII) durch Reaktion mit einer sekundären Base und Trifluoressigsäure, um das Zwischenprodukt der Formel (VIII) H-Cys(So₃H)-Cys(So₃H)-Glu-Tyr-Cys(SO₃H)-Cys-Asn-Pro-AIa- Cys-Thr-GIy-Cys-Tyr-OH TFA-Salz zu erhalten;
c) Durchführen einer nicht-oxidativen Cyclisierung am Zwischenprodukt (VIII), um rohes Linaclotid zu erhalten;
d) optional Reinigen des rohen Linaclotids, um gereinigtes Linaclotid zu erhalten;
e) optional Salzen des gereinigten Linaclotids mit Essigsäure, um das entsprechende Acetatsalz zu bilden.

2. Prozess nach Anspruch 1, wobei in Schritt b) die sekundäre Base ein sekundäres Amin ist, vorzugsweise DEA, Piperidin, Piperazin oder Morpholin, und Trifluoressigsäure mindestens 80 Volumen-% in Wasser beträgt.

3. Prozess nach Anspruch 1 oder 2, wobei Schritt c) durch intramolekulare nukleophile Substitution durchgeführt wird.

4. Prozess nach einem der vorstehenden Ansprüche, wobei Schritt c) in einem hydroalkoholischen Puffer bei einem pH-Wert zwischen 7 und 8 durchgeführt wird.

5. Prozess nach einem der vorstehenden Ansprüche, wobei in Schritt c) das rohe Linaclotid in wässriger Lösung erhalten wird.

6. Prozess nach einem der vorstehenden Ansprüche, wobei das Koppeln zwischen (II) und (III) und/oder das Koppeln zwischen [7-14] und (IV) und/oder das Koppeln zwischen [6-14] und (V) und/oder das Koppeln zwischen [5-14] und (VI) in Gegenwart von Ethylcyano-(hydroxyimino)-acetat oder dessen Derivaten oder 1,3-Dimethylbarbitursäurederivaten durchgeführt wird.

7. Prozess nach einem der vorstehenden Ansprüche, wobei das Kuppeln zwischen (II) und (III) und/oder das Kuppeln zwischen [7-14] und (IV) und/oder das Kuppeln zwischen [6-14] und (V) und/oder das Kuppeln zwischen [5-14] und (VI) in organischen polaren Lösungsmitteln, vorzugsweise ausgewählt aus DMF, NMP, ACN, durchgeführt wird.

8. Prozess nach einem der vorstehenden Ansprüche, wobei das Koppeln zwischen (II) und (III) und/oder das Koppeln zwischen [7-14] und (IV) und/oder das Koppeln zwischen [6-14] und (V) und/oder das Koppeln zwischen [5-14] und (VI) in einem Temperaturbereich zwischen - 10 °C und 35 °C, vorzugsweise zwischen 0 °C und 25 °C, durchgeführt wird.

9. Prozess nach einem der vorstehenden Ansprüche, wobei der Reinigungsschritt d) durch Umkehrphasen-Hochleistungsprobenverdrängung (RP-HPSD), Umkehrphasen-Hochleistungsflüssigkeitschromatographie (RP-HPLC) oder eine Kombination davon durchgeführt wird.

10. Prozess nach Anspruch 9, wobei die Elutionsphase aus einer wässrigen Lösung, einem polaren organischen Lösungsmittel oder einer Mischung davon besteht, wobei das polare organische Lösungsmittel vorzugsweise aus Trifluoressigsäure, Essigsäure, Acetonitril und einer Mischung davon ausgewählt ist, optional mit dem Zusatz eines Puffers, vorzugsweise eines Phosphorpuffers.

11. Prozess nach Anspruch 9 oder 10, wobei das gereinigte Linaclotid in Lösung vorliegt, vorzugsweise umfasst die Lösung Wasser, Essigsäure und Acetonitril.

12. Prozess nach einem der Ansprüche 9 bis 11, wobei Linaclotid eine HPLC-Reinheit gleich oder höher als 99,9 % und einen Gehalt an IMD-Linaclotid- und Cys-1-α-Keton-Linaclotid-Verunreinigungen von jeweils weniger als 100 ppm, vorzugsweise weniger als 50 ppm, besonders bevorzugt weniger als 40 ppm aufweist.

13. Prozess nach einem der vorstehenden Ansprüche, ferner umfassend einen Isolierungsschritt f) durch Gefriertrocknung ausgehend von einer hydroalkoholischen Lösung oder einer wässrigen Suspension.

14. Prozess nach Anspruch 13, wobei die hydroalkoholische Lösung tert-Butanol, Wasser, Essigsäure oder eine Mischung davon enthält.

15. Prozess nach Anspruch 13, wobei die wässrige Suspension durch Verdampfen der gereinigten Linaclotidlösung erhalten wird, vorzugsweise enthält die wässrige Suspension Essigsäure.

16. Verfahren zum Nachweis und zur Quantifizierung von IMD-Linaclotid und Cys-1-α-Keton im amorphen hochreinen Linaclotid oder seinem Acetatsalz, umfassend die Elution des Produkts durch eine Säule für Ionenpaarungschromatographie (IPC), die eine stationäre Phase aus Siliciumdioxid, die Alkylketten enthält, und eine Elutionsphase aufweist, die aus einer wässrigen Lösung, einem polaren organischen Lösungsmittel oder einer Mischung davon besteht, optional mit dem Zusatz eines Puffers, vorzugsweise eines Phosphorpuffers; wobei die Eluierungsphase ein Ionenpaarungsreagenz, vorzugsweise ein Heptansulfonatsalz, enthält.

17. Verfahren nach Anspruch 16, **dadurch gekennzeichnet, dass** die Alkylketten vom Octadecyl-, Octyl- oder Butyltyp (C18, C8 oder C4) sind, vorzugsweise C18.

18. Verfahren nach Anspruch 16 oder 17, **dadurch gekennzeichnet, dass** das polare organische Lösungsmittel ausgewählt ist aus Tetrahydrofuran, Dioxan, Dichlormethan, Methanol, Ethanol, n-Propanol, Isopropanol, Butanol, Pentan, Hexan, Toluol, Trifluoressigsäure, Acetonitril oder einer Mischung davon, vorzugsweise Trifluoressigsäure, Acetonitril oder einer Mischung davon.

19. Verfahren nach einem der Ansprüche 16 bis 18, **dadurch gekennzeichnet, dass** es unter den folgenden Betriebsbedingungen durchgeführt wird:
- Stationäre Phase: Unterstützung von Silikapartikeln, die C18-Alkylketten enthalten;
Eluant A: Phosphorsäurepuffer pH 6,2 + Natrium-1-heptansulfonat (Ionenpaarungsmittel)
Eluent B: ACN
wobei die folgende Gradientenelution angewendet wird:
% B: 7-7(2');7-15(19'); 15-60(25'),
und die folgenden Bedingungen implementiert sind: Durchfluss: 1,0 ml/min; Säule T: 30 °C, UV 220 nm.

20. Amorphes Linaclotid oder sein Acetatsalz, das eine chromatographische Reinheit von mindestens 99,9 % und eine Menge einer der folgenden Verunreinigungen von weniger als 100 ppm, vorzugsweise weniger als 50 ppm, besonders bevorzugt weniger als 40 ppm aufweist:
- IMD-Linaclotid (Verunreinigung 1;
- Cys-1-α-Keton (Verunreinigung 2);
wobei der Nachweis und die Quantifizierung von IMD-Linaclotid und Cys-1-α-Keton nach dem in einem der Ansprüche 16 bis 19 definierten Verfahren durchgeführt werden.

21. Amorphes hochreines Linaclotid oder sein Acetatsalz nach Anspruch 20, das einen Gehalt an Multimeren von 0,1 % Flächen-% oder weniger aufweist.

## Revendications

1. Procédé en phase liquide pour la production de linaclotide, de préférence sous la forme du sel acétate, qui comprend les étapes suivantes :
a1) le couplage d'un fragment de dipeptide B [7-8] de formule (II) Fmoc-Asn(Trt)-Pro-OH avec un fragment d'hexapeptide C [9-14] de formule (III) Fmoc-Ala-Cys[Ψ(Dmp,H)pro]-Thr(tBu)-Gly-Cys[Ψ(Dmp,H)pro]-Tyr(tBu)-OtBu pour obtenir le peptide [7-14] ;
a2) le couplage du peptide [7-14] avec un dérivé d'acide aminé de formule (IV) Fmoc-Cys(Trt)-OH en position 6 pour obtenir le peptide [6-14] ;
a3) le couplage du peptide [6-14] avec un dérivé d'acide aminé de formule (V) Fmoc-Cys(SO₃Na)-ONa en position 5 pour obtenir le peptide [5-14] ;
a4) le couplage du peptide [5-14] avec un fragment de tétrapeptide A [1-4] de formule (VI) Fmoc-Cys(SO₃Na)-Cys(SO₃Na)-Glu(tBu)-Tyr(tBu)-OH pour obtenir le peptide protégé de formule (VII) Fmoc-Cys(SO₃Na)-Cys(SO₃Na)-Glu(tBu)-Tyr(tBu)-Cys(SO₃Na)-Cys(Trt)-Asn(Trt)-Pro-Ala-Cys[Ψ(Dmp,H)pro]-Thr(tBu)-Gly-Cys[Ψ(Dmp,H)pro]-Tyr(tBu)-OtBu ;
b) la déprotection du composé (VII) par réaction avec une base secondaire et de l'acide trifluoroacétique pour obtenir l'intermédiaire de formule (VIII), le sel de TFA de H-Cys(So₃H)-Cys(So₃H)-Glu-Tyr-Cys(SO₃H)-Cys-Asn-Pro-Ala-Cys-Thr-Gly-Cys-Tyr-OH ;
c) la réalisation d'une cyclisation non oxydative sur l'intermédiaire (VIII) pour obtenir du Linaclotide brut ;
d) éventuellement, la purification du Linaclotide brut pour obtenir du Linaclotide purifié ;
e) éventuellement, la salification du Linaclotide purifié avec de l'acide acétique pour former le sel acétate correspondant.

2. Procédé selon la revendication 1, dans lequel, dans l'étape b), la base secondaire est une amine secondaire, de préférence la DEA, la pipéridine, la pipérazine ou la morpholine, et l'acide trifluoroacétique représente au moins 80 % en volume dans l'eau.

3. Procédé selon la revendication 1 ou 2, dans lequel l'étape c) est réalisée par substitution nucléophile intramoléculaire.

4. Procédé selon l'une quelconque des revendications précédentes, dans lequel l'étape c) est réalisée dans un tampon hydroalcoolique à un pH compris entre 7 et 8.

5. Procédé selon l'une quelconque des revendications précédentes, dans lequel, à l'étape c), le Linaclotide brut est obtenu en solution aqueuse.

6. Procédé selon l'une quelconque des revendications précédentes, dans lequel le couplage entre (II) et (III) et/ou le couplage entre [7-14] et (IV) et/ou le couplage entre [6-14] et (V) et/ou le couplage entre [5-14] et (VI) est réalisé en présence de cyano-(hydroxyimino)-acétate d'éthyle ou de ses dérivés, ou de dérivés de l'acide 1,3-diméthylbarbiturique.

7. Procédé selon l'une quelconque des revendications précédentes, dans lequel le couplage entre (II) et (III) et/ou le couplage entre [7-14] et (IV) et/ou le couplage entre [6-14] et (V) et/ou le couplage entre [5-14] et (VI) est réalisé dans des solvants polaires organiques, de préférence choisis parmi DMF, NMP, ACN.

8. Procédé selon l'une quelconque des revendications précédentes, dans lequel le couplage entre (II) et (III) et/ou le couplage entre [7-14] et (IV) et/ou le couplage entre [6-14] et (V) et/ou le couplage entre [5-14] et (VI) est réalisé dans une plage de température comprise entre -10 °C et 35 °C, de préférence entre 0 °C et 25 °C.

9. Procédé selon l'une quelconque des revendications précédentes, dans lequel l'étape de purification d) est réalisée par déplacement d'échantillon haute performance en phase inverse (RP-HPSD), chromatographie liquide haute performance en phase inverse (RP-HPLC) ou une combinaison de celles-ci.

10. Procédé selon la revendication 9, dans lequel la phase éluante est constituée d'une solution aqueuse, d'un solvant organique polaire ou d'un mélange de ceux-ci, de préférence le solvant organique polaire est choisi parmi l'acide trifluoroacétique, l'acide acétique, l'acétonitrile et un mélange de ceux-ci, éventuellement avec l'ajout d'un tampon, de préférence un tampon phosphorique.

11. Procédé selon la revendication 9 ou 10, dans lequel le Linaclotide purifié est en solution, de préférence ladite solution comprend de l'eau, de l'acide acétique et de l'acétonitrile.

12. Procédé selon l'une quelconque des revendications 9 à 11, dans lequel le Linaclotide a une pureté par HPLC égale ou supérieure à 99,9 % et une teneur en impuretés IMD-Linaclotide et Cys-1-α-cétone-Linaclotide chacune inférieure à 100 ppm, de préférence inférieure à 50 ppm, plus préférablement inférieure à 40 ppm.

13. Procédé selon l'une quelconque des revendications précédentes, comprenant en outre une étape d'isolement f) par lyophilisation à partir d'une solution hydroalcoolique ou d'une suspension aqueuse.

14. Procédé selon la revendication 13, dans lequel la solution hydroalcoolique contient du tert-butanol, de l'eau, de l'acide acétique ou un mélange de ceux-ci.

15. Procédé selon la revendication 13, dans lequel la suspension aqueuse est obtenue par évaporation de la solution de Linaclotide purifiée, de préférence la suspension aqueuse contient de l'acide acétique.

16. Procédé de détection et de quantification de l'IMD-Linaclotide et de la Cys-1-α-cétone dans le Linaclotide amorphe de haute pureté ou son sel acétate, comprenant l'élution du produit à travers une colonne de chromatographie par appariement d'ions (IPC), ayant une phase stationnaire de silice contenant des chaînes alkyle, et une phase éluante constituée d'une solution aqueuse, d'un solvant organique polaire, ou d'un mélange de ceux-ci, éventuellement avec l'ajout d'un tampon, de préférence un tampon phosphorique ; dans lequel la phase éluante contient un réactif d'appariement d'ions, de préférence un sel heptanesulfonate.

17. Procédé selon la revendication 16, **caractérisé en ce que** lesdites chaînes alkyle sont de type octadécyle, octyle ou butyle (C18, C8 ou C4), de préférence C18.

18. Procédé selon la revendication 16 ou 17, **caractérisé en ce que** ledit solvant organique polaire est choisi parmi le tétrahydrofurane, le dioxane, le dichlorométhane, le méthanol, l'éthanol, le n-propanol, l'isopropanol, le butanol, le pentane, l'hexane, le toluène, l'acide trifluoroacétique, l'acétonitrile ou un mélange de ceux-ci, de préférence l'acide trifluoroacétique, l'acétonitrile ou un mélange de ceux-ci.

19. Procédé selon l'une quelconque des revendications 16 à 18, **caractérisé en ce qu'**il est réalisé selon les conditions opératoires suivantes :
- phase stationnaire : particules de silice de support contenant des chaînes alkyle en C18 ;
Éluant A : tampon phosphorique pH 6,2 + 1-heptanesulfonate de sodium (agent d'appariement d'ions)
Éluant B : ACN
dans lequel le gradient d'élution suivant est appliqué :
% de B : 7-7(2') ; 7-15(19') ; 15-60(25'),
et les conditions suivantes sont mises en œuvre : Débit : 1,0 ml/min ; Colonne T : 30 °C, UV 220 nm.

20. Linaclotide amorphe ou son sel acétate ayant une pureté chromatographique égale ou supérieure à 99,9 % et une quantité de l'une quelconque des impuretés suivantes inférieure à 100 ppm, de préférence inférieure à 50 ppm, plus préférablement inférieure à 40 ppm :
- IMD-Linamolotide (impureté 1) ;
- Cys-1-α-cétone (impureté 2) ;
dans lequel la détection et la quantification de l'IMD-Linamolotide et de la Cys-1-α-cétone sont réalisées selon le procédé tel que défini dans l'une quelconque des revendications 16 à 19.

21. Linaclotide amorphe de haute pureté ou son sel acétate selon la revendication 20, ayant une teneur en multimères égale ou inférieure à 0,1 % en surface.
